(19) Europäisches Patentamt
European Patent Office
Office européen des brevets

(11) EP 4 670 739 A1

(12) **EUROPEAN PATENT APPLICATION**
published in accordance with Art. 153(4) EPC

(43) Date of publication:
**31.12.2025 Bulletin 2026/01**

(21) Application number: **24760604.9**

(22) Date of filing: **22.02.2024**

(51) International Patent Classification (IPC):
***A61K 47/64*** *(2017.01)* ***A61K 49/00*** *(2006.01)*
***A61K 41/00*** *(2020.01)*

(86) International application number:
**PCT/KR2024/002350**

(87) International publication number:
**WO 2024/177415 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.02.2023 KR 20230024648**

(71) Applicants:
- **Kytecbio Co., Ltd.**
  **Seoul 03759 (KR)**
- **Ewha University-Industry Collaboration Foundation**
  **Seoul 03760 (KR)**

(72) Inventors:
- **YOON, Ju Young**
  **Seoul 04428 (KR)**
- **KIM, Kwan Mook**
  **Incheon 22008 (KR)**
- **CHA, Sun Shin**
  **Seoul 03670 (KR)**
- **KIM, Gyoung Mi**
  **Seoul 07752 (KR)**
- **YANG, Mengyao**
  **Seoul 03721 (KR)**
- **SHIRBHATE, Mukesh Eknath**
  **Seoul 03721 (KR)**
- **KIM, Yu Jin**
  **Seoul 07993 (KR)**

(74) Representative: **Aseglio-Gianinet, Romina et al**
**Jacobacci & Partners S.p.A.**
**Corso Emilia, 8**
**10152 Torino (IT)**

# (54) ALBUMIN CONJUGATE, METHOD FOR PRODUCING SAME, AND COMPOSITION FOR TARGETING, DIAGNOSING, OR TREATING CANCER COMPRISING SAME

(57) Provided is an albumin conjugate represented by Formula 1:

Formula 1

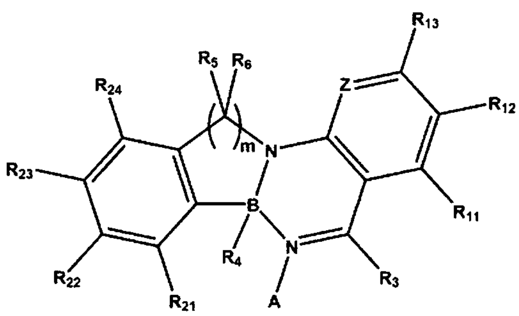

wherein, in Formula 1,
A may be a group other than an amine group of albumin, Z, $R_{11}$ to $R_{14}$, $R_{21}$ to $R_{24}$ and $R_3$ to $R_6$ and m are each as described in this specification.

EP 4 670 739 A1

FIG. 1

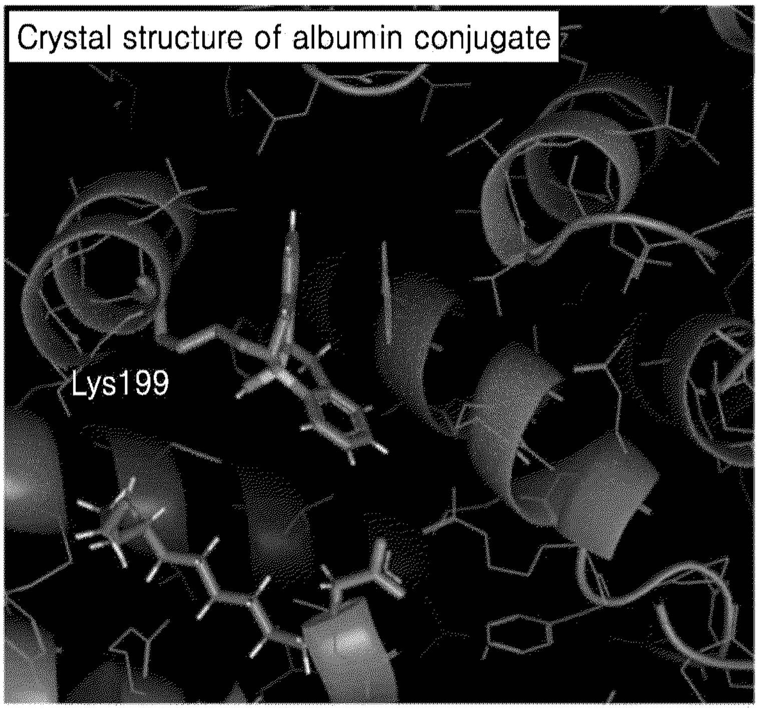

## Description

### Technical Field

The present disclosure relates to an albumin conjugate, a method of producing the same, and a composition including the same, the composition being for targeting, diagnosing, or treating cancer.

### Background Art

Human serum albumin (HSA) exists in human blood at about 35 g/L to about 55 g/L and can perform the function of delivering substances necessary for life activities. Albumin is a stable protein with a half-life of about 19 days. Cancer (cancer cells and/or cancer tissue) can receive nutrients necessary for growth through albumin. Cancer may have albumin receptors such as gp18, gp60, and SPARC to utilize albumin. Cancer can attract albumin through albumin receptors, whereby the albumin concentration in blood may decrease as the cancer grows. Utilizing such characteristics, research has continued on binding anticancer agents to albumin so as to deliver anticancer agents to cancer cells and/or cancer tissue.

Abraxane is a substance formed by simply mixing albumin with an anticancer agent (for example, paclitaxel). An anticancer agent simply mixed with albumin may initially be released excessively, and it is difficult to ensure reproducibility because the manner of binding between albumin and anticancer drugs cannot be controlled.

Maleimide can be applied to covalently bind albumin and an anticancer agent (for example, doxorubicin). Maleimide bound to an anticancer agent covalently binds with - SH at the cysteine-34 site of albumin. However, maleimide is unstable depending on pH, and the -SH located at the cysteine-34 site of albumin may be oxidized (for example, outside the body of a subject). Maleimide decomposes over time due to the instability of the ring structure. Therefore, there is a need for stable binding between albumin and anticancer agents.

### Disclosure of Invention

### Technical Problem

One aspect is to provide a conjugate of an anticancer agent and albumin, the conjugate with high reproducibility and high stability.

One aspect is to provide albumin conjugates that can be used for cancer targeting, diagnosis or therapy.

Another aspect is to provide a method of producing the albumin conjugate.

Another aspect is to provide a composition including the albumin conjugate, the composition for targeting, diagnosing or treating cancer.

Another aspect is to provide a composition including a boron-containing compound, the composition for targeting, diagnosing, or treating cancer.

### Solution to Problem

According to an aspect, an albumin conjugate represented by Formula 1 is provided:

Formula 1

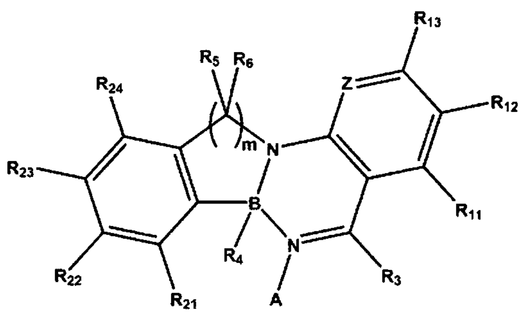

**wherein,** in Formula 1,

A may be a group other than an amine group of albumin,

Z may be N, O, or $C(R_{14})$,

$R_{11}$ to $R_{14}$, R21 to R24 and $R_3$ to $R_6$ may each independently be selected from hydrogen, deuterium, -F, -Cl, -Br, -I, a hydroxyl group, a cyano group, a nitro group, a formyl group, a carboxyl group, an amidino group, a hydrazino group, a hydrazono group, a substituted or unsubstituted $C_1$-$C_{10}$ alkyl group, a substituted or unsubstituted $C_2$-$C_{10}$ alkenyl group, a substituted or unsubstituted $C_2$-$C_{10}$ alkynyl group, a substituted or unsubstituted $C_1$-$C_{20}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ **heterocycloalkenyl** group, a substituted or unsubstituted $C_6$-$C_{20}$ aryl group, a substituted or unsubstituted $C_6$-$C_{20}$ aryloxy group, a substituted or unsubstituted $C_6$-$C_{20}$ arylthio group, a substituted or unsubstituted $C_1$-$C_{20}$ heteroaryl group, $-Si(Q_1)(Q_2)(Q_3)$, $-N(Q_1)(Q_2)$, - $B(Q_1)(Q_2)$, $-C(=O)(Q_1)$, $-S(=O)_2(Q_1)$, and $-P(=O)(Q_1)(Q_2)$,

optionally, i) $R_{11}$ and $R_{12}$, ii) $R_{12}$ and $R_{13}$, iii) $R_{13}$ and $R_{14}$, iv) $R_{21}$ and $R_{22}$, v) $R_{22}$ and $R_{23}$, vi) $R_{23}$ and R24, or vii) any combination thereof may be bound to each other to form a substituted or unsubstituted $C_3$-$C_{10}$ carbocyclic group or a substituted or unsubstituted $C_1$-$C_{10}$ heterocyclic group,

m may be an integer from 1 to 5, and

$Q_1$ to $Q_3$ may each independently be selected from

hydrogen, deuterium, -F, -Cl, -Br, -I, a hydroxyl group, a cyano group, a nitro group, an amidino group, a hydrazino group, a hydrazono group; and

a $C_1$-$C_{10}$ alkyl group, a $C_2$-$C_{10}$ alkenyl group, a $C_2$-$C_{10}$ alkynyl group, a $C_1$-$C_{10}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{20}$ aryl group, and a $C_1$-$C_{20}$ heteroaryl group, each unsubstituted or substituted with hydrogen, deuterium, -F, -Cl, -Br, -I, a hydroxyl group, a cyano group, a nitro group, an amidino group, a hydrazino group, a hydrazono group, or any combination thereof.

According to another aspect, a method of producing the albumin conjugate is provided, including reacting albumin with a boron-containing compound represented by Formula 2:

Formula 2

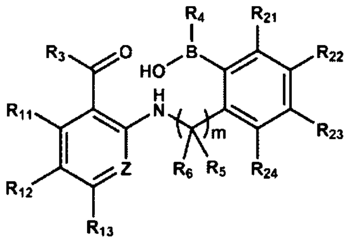

wherein, in Formula 2,

Z may be N, O, or $C(R_{14})$,

$R_{11}$ **to** $R_{14}$, R21 to $R_{24}$ and $R_3$ to $R_6$ may each independently be selected from hydrogen, deuterium, -F, -Cl, -Br, -I, a hydroxyl group, a cyano group, a nitro group, a formyl group, a carboxyl group, an amidino group, a hydrazino group, a hydrazono group, a substituted or unsubstituted $C_1$-$C_{10}$ alkyl group, a substituted or unsubstituted $C_2$-$C_{10}$ alkenyl group, a substituted or unsubstituted $C_2$-$C_{10}$ alkynyl group, a substituted or unsubstituted $C_1$-$C_{20}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{20}$ aryl group, a substituted or unsubstituted $C_6$-$C_{20}$ aryloxy group, a substituted or unsubstituted $C_6$-$C_{20}$ arylthio group, a substituted or unsubstituted $C_1$-$C_{20}$ heteroaryl group, $-Si(Q_1)(Q_2)(Q_3)$, $-N(Q_1)(Q_2)$, - $B(Q_1)(Q_2)$, $-C(=O)(Q_1)$, $-S(=O)_2(Q_1)$, and $-P(=O)(Q_1)(Q_2)$,

optionally, i) $R_{11}$ and $R_{12}$, ii) $R_{12}$ and $R_{13}$, iii) $R_{13}$ and $R_{14}$, iv) $R_{21}$ and $R_{22}$, v) $R_{22}$ and $R_{23}$, vi) $R_{23}$ and R24, or vii) any combination thereof may be bound to each other to form a substituted or unsubstituted $C_3$-$C_{10}$ carbocyclic group or a substituted or unsubstituted $C_1$-$C_{10}$ heterocyclic group,

m may be an integer from 1 to 5, and

$Q_1$ to $Q_3$ may each independently be selected from

hydrogen, deuterium, -F, -Cl, -Br, -I, a hydroxyl group, a cyano group, a nitro group, an amidino group, a hydrazino group, a hydrazono group; and

a $C_1$-$C_{10}$ alkyl group, a $C_2$-$C_{10}$ alkenyl group, a $C_2$-$C_{10}$ alkynyl group, a $C_1$-$C_{10}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{20}$

aryl group, and a $C_1$-$C_{20}$ heteroaryl group, each unsubstituted or substituted with hydrogen, deuterium, -F, -Cl, -Br, -I, a hydroxyl group, a cyano group, a nitro group, an amidino group, a hydrazino group, a hydrazono group, or any combination thereof.

According to another aspect, provided is a composition for targeting, diagnosing or treating cancer including the albumin conjugate or the boron-containing compound.

## Advantageous Effects of Invention

The boron-containing compound represented by Formula 2 described above can stably bind to albumin to form an albumin conjugate represented by Formula 1. The albumin conjugate can selectively accumulate in cancer within a subject, thereby targeting cancer cells and/or cancer tissue. The albumin conjugate may have fluorescent properties, which may enable visualization of cancer tissue (fluorescence imaging). When the albumin conjugate is irradiated with light, reactive oxygen species (ROS) are generated, and cancer can be treated.

## Brief Description of Drawings

FIG. 1 illustrates an X-ray crystal structure of an albumin conjugate according to an embodiment of the present disclosure.

FIG. 2 is a diagram showing changes in fluorescence intensity measured while increasing the concentration of albumin in a boron-containing compound, and the dissociation constant calculated based on the changes.

FIGS. 3A to 3C illustrate fluorescence intensities generated by irradiating light onto an albumin conjugate according to an embodiment of the present disclosure.

FIGS. 4A to 4C illustrate fluorescence intensities generated by irradiating light onto a boron-containing compound.

FIGS. 5A and 5B illustrate fluorescence intensities generated by irradiating light in the absence of an albumin conjugate and a boron-containing compound.

FIG. 6 is a diagram showing the results shown in FIGS. 3A to 3C, 4A to 4C, 5A, and 5B as differences in fluorescence intensity at a specific wavelength over time.

FIGS. 7A to 7D are diagrams showing differences in fluorescence intensities according to concentrations of lysine mixed with a boron-containing compound.

FIG. 8 is a diagram showing the results of observing fluorescence characteristics upon injecting albumin conjugates using bovine serum albumin and human serum albumin, respectively, into a subject.

FIGS. 9A, 9B, and 10A to 10C are diagrams showing the results of evaluating the cancer treatment capabilities of albumin conjugates according to an embodiment of the present disclosure.

## Best Mode for Carrying out the Invention

## Mode for the Invention

All technical terms used in this specification, unless otherwise defined, have the same meaning as commonly understood by one of ordinary skill in the art. Additionally, the numerical values described in this specification are considered to include the meaning of "about" even if not specified.

The term "include" in this specification is used to indicate the addition and/or interposition of other components, unless specifically stated to the contrary, rather than the exclusion of other components.

The term "any combination" as used herein refers to a mixture or combination of one or more of the described components.

The term "interaction" as used herein can be direct or indirect, can involve direct binding or can involve indirect binding, and the binding can also be mediated by another molecule.

In this specification, a substituent is derived by exchanging one or more hydrogens in an unsubstituted mother group with another atom or functional group. Unless otherwise stated, when a functional group is considered to be "substituted", it means that the functional group is substituted with one or more substituents selected from deuterium, -F, -Cl, -Br, -I, a hydroxyl group, a cyano group, a nitro group, an amidino group, a hydrazino group, a hydrazono group, an alkyl group having 1 to 40 carbon atoms, an alkenyl group having 2 to 40 carbon atoms, an alkynyl group having 2 to 40 carbon atoms, a cycloalkyl group having 3 to 40 carbon atoms, a cycloalkenyl group having 3 to 40 carbon atoms, an aryl group having 6 to 40 carbon atoms, and a heteroaryl group having 3 to 40 carbon atoms.

When a functional group is described as being "optionally substituted," it means that the functional group can be

substituted with the substituents described above.

In this specification, a and b in "a to b carbon atoms" each refer to the number of carbon atoms in a specific functional group. That is, the functional group may include carbon atoms from a to b. For example, "an alkylene group having 1 to 4 carbon atoms" refers to an alkylene group having 1 to 4 carbon atoms, i.e., $-CH_2-$, $-CH_2CH_2-$, $-CH_2CH_2CH_2-$, $-(CH_3)_2C-$, $-CH_2CH_2CH_2CH_2-$, $-CH_2CH_2CH(CH_3)-$, and $-(CH_3)_2C-$.

The term "alkyl" as used herein refers to a branched or unbranched aliphatic hydrocarbon. In an embodiment, the alkyl group may be substituted or unsubstituted. An alkyl group may include, but are not necessarily limited to, a methyl group, an ethyl group, a propyl group, an isopropyl group, a butyl group, an isobutyl group, a tert-butyl group, a pentyl group, a hexyl group, a cyclopropyl group, a cyclopentyl group, a cyclohexyl group, a cycloheptyl group, and the like, each of which may be optionally substituted or unsubstituted. In an embodiment, the alkyl group may have 1 to 6 carbon atoms. For example, an alkyl group having 1 to 6 carbon atoms may be, but is not necessarily limited to, methyl, ethyl, propyl, isopropyl, butyl, iso-butyl, sec-butyl, pentyl, 3-pentyl, hexyl, and the like.

The term "alkenyl" as used herein refers to a branched or unbranched hydrocarbon having at least one carbon-carbon double bond. Non-limiting examples of the alkenyl group include vinyl, allyl, butenyl, isopropenyl, or isobutenyl.

The term "alkynyl" as used herein refers to a branched or unbranched hydrocarbon having at least one carbon-carbon triple bond.

The term "alkoxy" as used herein refers to a form in which an alkyl group as defined above is bound to an oxygen atom, which may include a $C_1$-$C_{20}$ alkoxy(group), and may include, but is not limited to, methoxy, ethoxy, propoxy, butoxy, pentoxy, hexyloxy, hepyloxy, octyloxy, nonyloxy, decyloxy, undecyloxy, dodecyloxy, tridecyloxy, tetradecyloxy, pentadecyloxy, hexadecyloxy, heptadecyloxy, octadecyloxy, nonadecyloxy, eicosanyloxy, or all possible isomers thereof.

The term "cycloalkyl" as used herein is in the form of a monovalent functional group having a saturated hydrocarbon ring, and may include $C_3$-$C_8$ cycloalkyl (group), for example, may include cyclopropyl, cyclobutyl, cyclopentyl, cyclohexyl, cycloheptyl, cyclooctyl, or all possible isomers thereof, but is not limited thereto.

The term "aryl" as used herein refers to an aromatic ring whose ring skeleton contains only carbon, a ring system (i.e., two or more fused rings sharing two adjacent carbon atoms), or a ring in which a plurality of aromatic rings are connected to each other by a single bond, -O-, -S-, -C(=O)-, $-S(=O)_2-$, -Si(Ra)(Rb)- (wherein Ra and Rb are each independently an alkyl group having 1 to 10 carbon atoms), an alkylene group having 1 to 10 carbon atoms unsubstituted or substituted with halogen, or -C(=O)-NH-. When the aryl group is a ring system, each ring in the system may be aromatic. For example, the aryl group includes, but is not limited to, a phenyl group, a biphenyl group, a naphthyl group, a phenanthrenyl group, a naphthacenyl group, and the like. The aryl group may be substituted or unsubstituted.

The term "heteroaryl" as used herein refers to a monocyclic or bicyclic organic compound containing one or more heteroatoms selected from N, O, P or S, with the remaining ring atoms being carbon. The heteroaryl group has no particular limitation on the number of carbon atoms, but may have 2 to 60 carbon atoms. The heteroaryl group may be, for example, a substituted or unsubstituted imidazolyl group, a substituted or unsubstituted triazolyl group, a substituted or unsubstituted tetrazolyl group, a substituted or unsubstituted oxadiazolyl group, a substituted or unsubstituted oxatriazolyl group, a substituted or unsubstituted thiatriazolyl group, a substituted or unsubstituted benzimidazolyl group, a substituted or unsubstituted benzotriazolyl group, a substituted or unsubstituted pyridinyl group, a substituted or unsubstituted pyrimidinyl group, a substituted or unsubstituted triazinyl group, a substituted or unsubstituted pyrazinyl group, a substituted or unsubstituted pyridazinyl group, a substituted or unsubstituted quinoline, a substituted or unsubstituted isoquinoline, a substituted or unsubstituted phthalazine, a substituted or unsubstituted naphpyridine, a substituted or unsubstituted quinoxaline, a substituted or unsubstituted quinazoline, a substituted or unsubstituted acridine, a substituted or unsubstituted phenanthroline, a substituted or unsubstituted phenazine, or a combination thereof.

The albumin conjugate according to one aspect is represented by Formula 1:

Formula 1

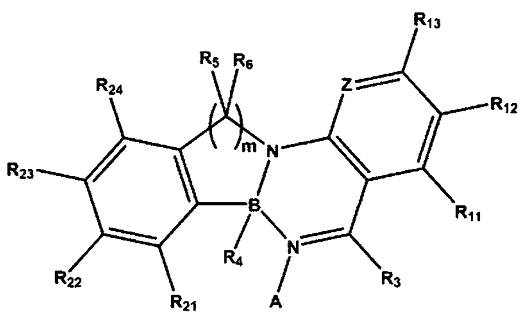

wherein, in Formula 1, A may be a group excluding an amine group in albumin. According to an embodiment, the amine

group may be an amine group included in a side chain of lysine represented by Formula 3. According to another embodiment, the amine group may be an amine group included in the main chain of lysine represented by Formula 3.

Formula 3

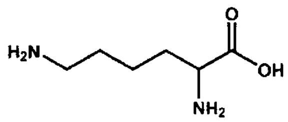

**In an embodiment,** the amine group may be located at the SS1 site of the albumin. For example, in Formula 1, A may be any group except the amine group of lysine located at the SS1 site in the albumin. The lysine located at the SS1 site of the albumin may be referred to as lysine-199 (Lysine-199 or Lys-199).

According to an embodiment, the albumin conjugate may include human serum albumin (HSA) or bovine serum albumin (BSA). That is, the albumin may be human serum albumin (HSA) or bovine serum albumin (BSA).

In Formula 1, Z may be N, O, or $C(R_{14})$, and a description of $R_{14}$ is provided below. In an embodiment, Z may be, but is not limited to, $C(R_{14})$.

$R_{11}$ to $R_{14}$, $R_{21}$ to $R_{24}$ and $R_3$ to $R_6$ in Formula 1 may each independently be selected from hydrogen, deuterium, -F, -Cl, -Br, -I, a hydroxyl group, a cyano group, a nitro group, a formyl group, a carboxyl group, an amidino group, a hydrazino group, a hydrazono group, a substituted or unsubstituted $C_1$-$C_{10}$ alkyl group, a substituted or unsubstituted $C_2$-$C_{10}$ alkenyl group, a substituted or unsubstituted $C_2$-$C_{10}$ alkynyl group, a substituted or unsubstituted $C_1$-$C_{20}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{20}$ aryl group, a substituted or unsubstituted $C_6$-$C_{20}$ aryloxy group, a substituted or unsubstituted $C_6$-$C_{20}$ arylthio group, a substituted or unsubstituted $C_1$-$C_{20}$ heteroaryl group, $-Si(Q_1)(Q_2)(Q_3)$, $-N(Q_1)(Q_2)$, $-B(Q_1)(Q_2)$, $-C(=O)(Q_1)$, $-S(=O)_2(Q_1)$, and $-P(=O)(Q_1)(Q_2)$, and descriptions of $Q_1$ to $Q_3$ are described below.

**In Formula** 1, optionally, i) $R_{11}$ and $R_{12}$, ii) $R_{12}$ and $R_{13}$, iii) $R_{13}$ and $R_{14}$, iv) $R_{21}$ and $R_{22}$, v) $R_{22}$ and $R_{23}$, vi) $R_{23}$ and $R_{24}$, or vii) any combination thereof may be bound to each other to form a substituted or unsubstituted $C_3$-$C_{10}$ carbocyclic group or a substituted or unsubstituted $C_1$-$C_{10}$ heterocyclic group. According to an embodiment, in Formula 1, $R_{12}$ and $R_{13}$ may be bound to each other to form a substituted or unsubstituted $C_3$-$C_{10}$ carbocyclic group or a substituted or unsubstituted $C_1$-$C_{10}$ heterocyclic group, but are not limited thereto.

According to an embodiment, in Formula 1, at least one of $R_{11}$ to $R_{14}$ may be selected from -F, -Cl, -Br, -I, a substituted or unsubstituted $C_1$-$C_{10}$ alkyl group, a substituted or unsubstituted $C_2$-$C_{10}$ alkenyl group, and a substituted or unsubstituted $C_1$-$C_{20}$ alkoxy group, but is not limited thereto.

According to an embodiment, in Formula 1, each of $R_{21}$ to $R_{24}$ may be hydrogen, but is not limited thereto.

According to an embodiment, in Formula 1, $R_3$ may be selected from hydrogen, deuterium, and a substituted or unsubstituted $C_6$-$C_{20}$ aryl group, but is not limited thereto.

According to an embodiment, in Formula 1, $R_4$ may be a hydroxyl group, but is not limited thereto.

According to an embodiment, in Formula 1, each of $R_3$ to $R_6$ may be hydrogen, but is not limited thereto.

In Formula 1, m may be an integer selected from 1 to 5. In an embodiment, m may be 1, but is not limited thereto.

$Q_1$ to $Q_3$ may each independently be selected from

hydrogen, deuterium, -F, -Cl, -Br, -I, a hydroxyl group, a cyano group, a nitro group, an amidino group, a hydrazino group, a hydrazono group; and

a $C_1$-$C_{10}$ alkyl group, a $C_2$-$C_{10}$ alkenyl group, a $C_2$-$C_{10}$ alkynyl group, a $C_1$-$C_{10}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{20}$ aryl group and a $C_1$-$C_{20}$ heteroaryl group, each unsubstituted or substituted with hydrogen, deuterium, -F, -Cl, -Br, -I, a hydroxyl group, a cyano group, a nitro group, an amidino group, a hydrazino group, a hydrazono group, or any combination thereof.

According to an embodiment, the albumin conjugate may be any one of albumin conjugates B-A1 to B-A5, but is not limited thereto:

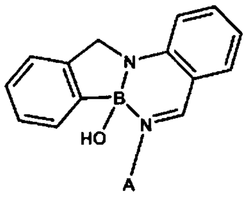

B-A1

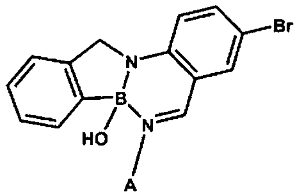

B-A2

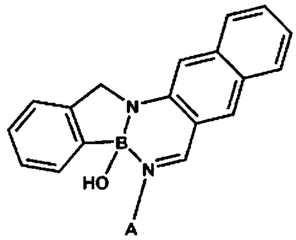

B-A3

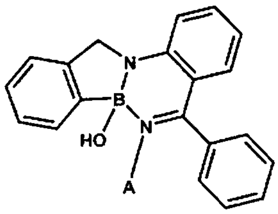

B-A4

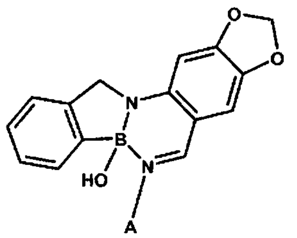

B-A5

A in albumin conjugate B-A1 to albumin conjugate B-A5 may be the same as described herein.

In an embodiment, the albumin conjugate may have fluorescent properties.

According to an embodiment, the albumin conjugate may generate reactive oxygen species under light irradiation.

According to another aspect, a method of producing the albumin conjugate is provided, including reacting albumin with a boron-containing compound represented by Formula 2:

Formula 2

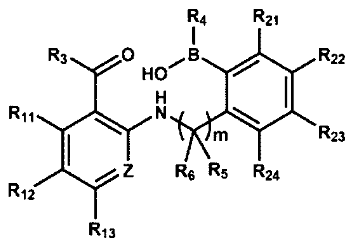

wherein, in Formula 2,

Z may be N, O, or C($R_{14}$),

$R_{11}$ **to** $R_{14}$, $R_{21}$ to $R_{24}$ and $R_3$ to $R_6$ may each independently be selected from hydrogen, deuterium, -F, -Cl, -Br, -I, a hydroxyl group, a cyano group, a nitro group, a formyl group, a carboxyl group, an amidino group, a hydrazino group, a hydrazono group, a substituted or unsubstituted $C_1$-$C_{10}$ alkyl group, a substituted or unsubstituted $C_2$-$C_{10}$ alkenyl group, a substituted or unsubstituted $C_2$-$C_{10}$ alkynyl group, a substituted or unsubstituted $C_1$-$C_{20}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{20}$ aryl group, a substituted or unsubstituted $C_6$-$C_{20}$ aryloxy group, a substituted or unsubstituted $C_6$-$C_{20}$ arylthio group, a substituted or unsubstituted $C_1$-$C_{20}$ heteroaryl group, -Si($Q_1$)($Q_2$)($Q_3$), -N($Q_1$)($Q_2$), - B($Q_1$)($Q_2$), -C(=O)($Q_1$), -S(=O)$_2$($Q_1$), and -P(=O)($Q_1$)($Q_2$),

optionally, i) $R_{11}$ and $R_{12}$, ii) $R_{12}$ and $R_{13}$, iii) $R_{13}$ and $R_{14}$, iv) $R_{21}$ and $R_{22}$, v) $R_{22}$ and $R_{23}$, vi) $R_{23}$ and $R_{24}$, or vii) any combination thereof may be bound to each other to form a substituted or unsubstituted $C_3$-$C_{10}$ carbocyclic group or a substituted or unsubstituted $C_1$-$C_{10}$ heterocyclic group,

m may be an integer from 1 to 5, and

$Q_1$ to $Q_3$ may each independently be selected from

hydrogen, deuterium, -F, -Cl, -Br, -I, a hydroxyl group, a cyano group, a nitro group, an amidino group, a hydrazino group, a hydrazono group; and

a $C_1$-$C_{10}$ alkyl group, a $C_2$-$C_{10}$ alkenyl group, a $C_2$-$C_{10}$ alkynyl group, a $C_1$-$C_{10}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{20}$

aryl group, and a $C_1$-$C_{20}$ heteroaryl group, each unsubstituted or substituted with hydrogen, deuterium, -F, -Cl, -Br, -I, a hydroxyl group, a cyano group, a nitro group, an amidino group, a hydrazino group, a hydrazono group, or any combination thereof.

Z, $R_{11}$ to $R_{14}$, $R_{21}$ to $R_{24}$ and $R_3$ to $R_6$ and m in Formula 2 are respectively as described in connection with Z, $R_{11}$ to R14, $R_{21}$ to $R_{24}$ and $R_3$ to $R_3$ and m in Formula 1.

According to an embodiment, the boron-containing compound may be one of compounds B1 to B5:

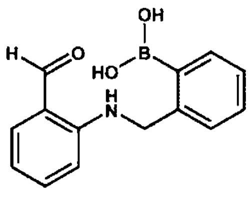
B1

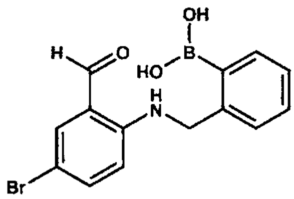
B2

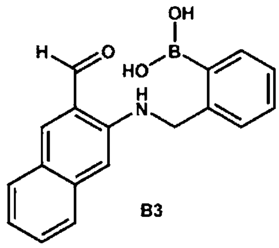
B3

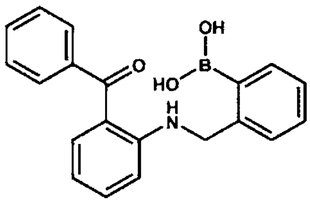
B4

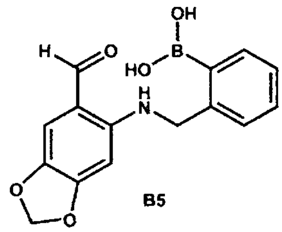
B5

The method of producing the albumin conjugate may include mixing the boron-containing compound represented by Formula 2 and HSA.

According to an embodiment, in Formula 2, boron (B) may react with albumin to form four-coordinates. In some embodiments, tri-coordinate boron (B) may become tetra-coordinate boron (B) by reacting with albumin.

In an embodiment, the reacting the boron-containing compound with albumin may be performed outside the body of the subject.

In an embodiment, the reacting the boron-containing compound with albumin may be performed in the blood of the subject. For example, the albumin is present in the blood of the subject, and by administering the boron-containing compound to the subject, the boron-containing compound and the albumin may react. That is, the albumin conjugate may be produced within the body of the subject.

According to another aspect, provided is a composition for targeting, diagnosing or treating cancer, the composition including the albumin conjugate or the boron-containing compound.

Examples of the cancer are, but are not limited to, breast cancer, kidney cancer, testicular cancer, prostate cancer, ovarian cancer, uterine cancer, cervical cancer, vaginal cancer, fallopian tube cancer, rectal cancer, lung cancer, stomach cancer, liver cancer, esophageal cancer, small intestine cancer, pancreatic cancer, and oral cancer.

In an embodiment, the albumin conjugate or the boron-containing compound may have fluorescence imaging properties. For example, the albumin conjugate or the boron-containing compound may emit fluorescence to visualize cancer (cancer cells and/or cancer tissue).

According to an embodiment, the albumin conjugate or the boron-containing compound may have reactive oxygen species-generating properties. For example, the albumin conjugate or the boron-containing compound may generate reactive oxygen species under light irradiation.

The carbonyl group of the boron-containing compound represented by Formula 2 may react with the amine group of lysine (Lysine-199 or Lys-199) located at the SS1 site of albumin to form an imine bond. Thereafter, the nitrogen of the imine bond and the boron of the boron-containing compound may be bound to each other to form the albumin conjugate represented by Formula 1.

Since the albumin conjugate includes the albumin-derived group, the albumin conjugate may selectively accumulate in cancer (cancer cells and/or cancer tissues) having an albumin receptor. Therefore, the albumin conjugate may be effectively delivered to and target cancer.

The albumin conjugate may have fluorescent properties because the albumin conjugate has a fluorescent chromophore including an imine bond and a nitrogen-boron-nitrogen (NBN) group. Thus, the albumin conjugate may visualize and/or perform fluorescence-imaging cancer.

Since the albumin conjugate may generate reactive oxygen species under light irradiation, the photodynamically

utilized albumin conjugate may treat cancer.

In addition, the albumin conjugate may be easily manufactured because the albumin conjugate is produced relatively quickly by simply mixing the boron-containing compound with albumin. Therefore, since albumin exists in the blood of a subject (for example, HSA exists in human blood at about 35 g/L to about 55 g/L), when the boron-containing compound represented by Formula 2 is administered to a subject, the albumin conjugate represented by Formula 1 may be produced relatively quickly and easily in the subject.

FIG. 1 illustrates an X-ray crystal structure of an albumin conjugate according to an embodiment of the present disclosure.

Referring to FIG. 1, it can be seen that the boron-containing compound represented by Formula 2 covalently binds with an amine group contained in lysine (Lys-199) located at the SS1 site of albumin. That is, compared to a conjugate that is unable to be formed when -SH of the cysteine-34 site in albumin is oxidized, the albumin conjugate according to the present disclosure may be stably formed by stably binding the boron-containing compound and albumin to each other. For example, the albumin conjugate according to the present disclosure is stable at about pH 3 to 12, and the SS1 site of albumin is not oxidized even when exposed to air.

**Synthesis Example 1 (Synthesis of Albumin Conjugate)**

The boron-containing compound represented by Formula 2 and human serum albumin were mixed 1:1 in a phosphate-buffered saline (PBS) buffer solution (pH 7.4) to make a 200 μM solution, and stirred. This solution was stored in a refrigerator or at room temperature to produce an albumin conjugate represented by Formula 1 according to reaction scheme 1:

Reaction Scheme 1

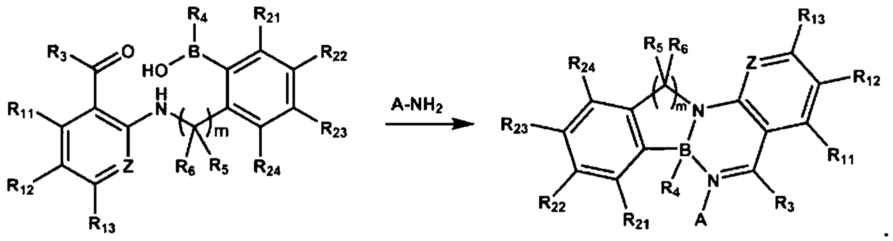


In Reaction Scheme 1,

A-$NH_2$ is albumin,
$NH_2$ is the amine group of lysine located at the SS1 site in albumin, and
A is any group except the amine group of lysine (Lysine-199 or Lys-199) located at the SS1 site in albumin.

According to Reaction Scheme 1, as the tri-coordinate boron included in the boron-containing compound changes into tetra-coordinate boron included in the albumin conjugate, the albumin conjugate may have fluorescent properties. In particular, when a boron-containing compound binds to the amine group of lysine (Lysine-199 or Lys-199) in albumin, an imine bond and a nitrogen-boron-nitrogen (NBN) group are formed, thereby generating a fluorescent chromophore. That is, the boron-containing compound represented by Formula 2 may include a carbonyl group (C=O) capable of forming an imine bond by reacting with an amine group and a boronic acid group (-$B(OH)_2$) capable of generating a four-coordinate fluorescent chromophore by reacting with an imine bond, at appropriate positions.

**Synthesis Example 2-1 (Synthesis of boron-containing compound B1)**

Compound B1 may be produced by using a method known in the art, and for example, compound B1 can be synthesized using the method of Reaction Scheme 2-1, but is not limited thereto.

Reaction Scheme 2-1

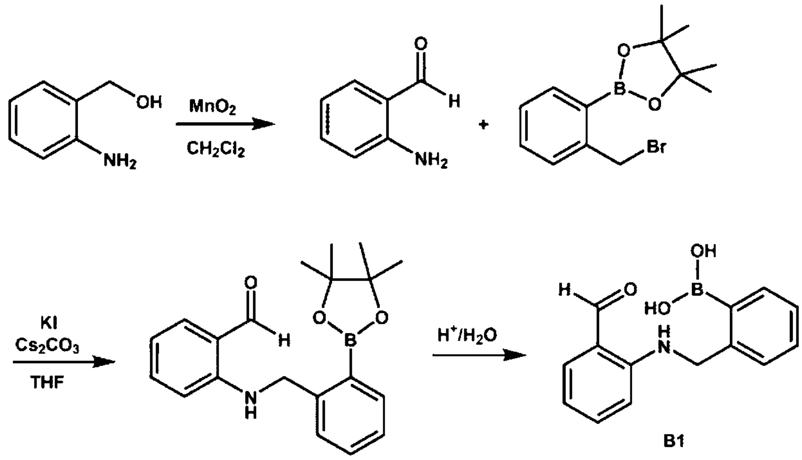


B1

**<u>Synthesis Example 2-2 (Synthesis of boron-containing compound</u>** <u>B2)</u>

Compound B2 may be produced by using a method known in the art, and for example, compound B2 can be synthesized using the method of Reaction Scheme 2-2, but is not limited thereto.

Reaction Scheme 2-2

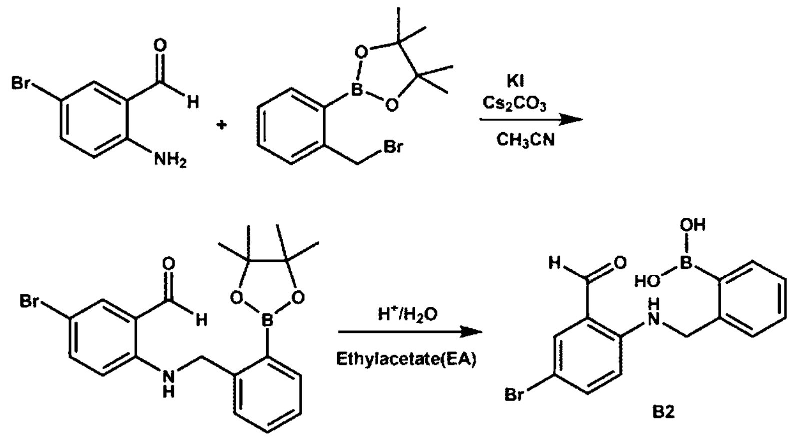


B2

**<u>Synthesis Example 2-3 (Synthesis of boron-containing compound</u>** <u>B3)</u>

Compound B3 may be produced by using a method known in the art, and for example, compound B3 can be synthesized using the method of Reaction Scheme 2-3, but is not limited thereto.

Reaction Scheme 2-3

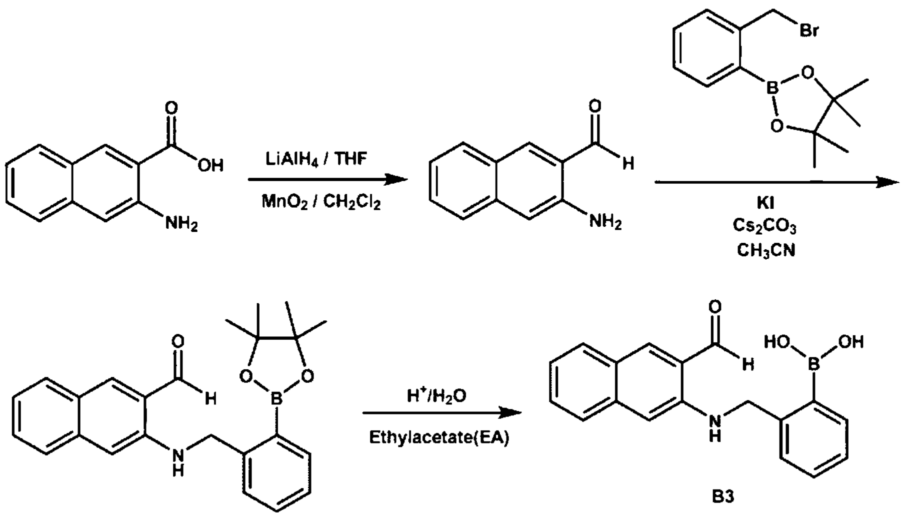


B3

**Synthesis Example 2-4 (Synthesis of boron-containing compound B4)**

Compound B4 may be produced by using a method known in the art, and for example, compound B4 can be synthesized using the method of Reaction Scheme 2-4, but is not limited thereto.

Reaction Scheme 2-4

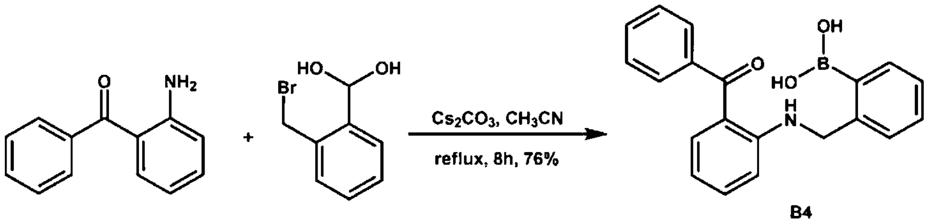


B4

**Synthesis Example 2-5 (Synthesis of boron-containing compound B5)**

Compound B5 may be produced by using a method known in the art, and for example, compound B5 can be synthesized using the method of Reaction Scheme 2-5, but is not limited thereto. EtOH in Reaction Scheme 2-5 is ethanol.

Reaction Scheme 2-5

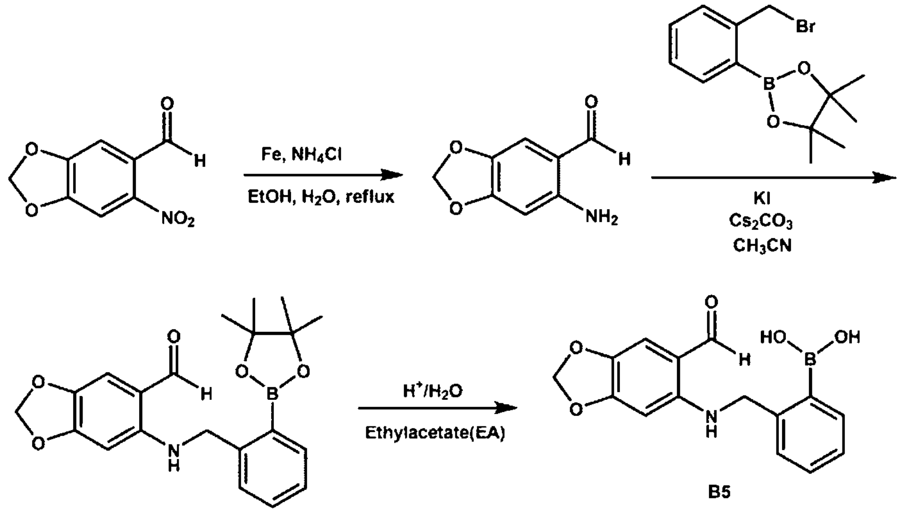


B5

## Evaluation Example 1 (Binding Ability Evaluation)

100 nM boron-containing compound B1 and albumin (HSA) were mixed in 95 vol% PBS buffer and 5 vol% dimethyl sulfoxide (DMSO) and stirred for 2 hours. The change in fluorescence intensity (F.I.) was measured while increasing the concentration of the albumin, and the dissociation constant ($K_d$) calculated based thereon is shown in FIG. 2.

(a) of FIG. 2 shows the change in the fluorescence intensity (F.I.) according to the concentration ratio of albumin to 100 nM boron-containing compound B1. For example, 0:1 represents the case where 100 nM albumin (HSA) was used without using a boron-containing compound, 1:0 represents the case where 100 nM boron-containing compound B1 was used and no albumin (HSA) was used, 1:2 represents the case where 100 nM boron-containing compound B1 and 200 nM albumin (HSA) were used, and 1:16 represents the case where 100 nM boron-containing compound B1 and 1600 nM albumin (HSA) were used.
(b) of FIG. 2 shows the calculation of the dissociation constant ($K_d$) using a fitting program based on the change in the fluorescence intensity (F.I.) at 530 nm.

The dissociation constants ($K_d$) of boron-containing compounds B2 to B5 were calculated in the same method as used to produce boron-containing compound B1. Results thereof are shown in Table 1 below.

[Table 1]

| Boron-containing compound | B1 | B2 | B3 | B4 | B5 |
|---|---|---|---|---|---|
| Albumin conjugate | B-A1 | B-A2 | B-A3 | B-A4 | B-A5 |
| Dissociation constant ($\mu$M) | 0.185 | 0.383 | 2.48 | 2.22 | 0.571 |

From Table 1, it can be seen that the dissociation constant ($K_d$) of each of albumin conjugate B-A1 to albumin conjugate B-A5 is about 0.1 $\mu$M to about 2.5 $\mu$M. Therefore, it can be seen that each of albumin conjugates B-A1 to albumin conjugate B-A5, which have relatively small dissociation constants, have excellent binding abilities. This may be because each of boron-containing compound B1 to boron-containing compound B5 not only binds to albumin via van der Waals bonds, but also covalently binds to lysine (Lysine-199 or Lys-199) located at the SS1 site of albumin. However, the dissociation constant of the albumin conjugate according to Formula 1 is not limited to the range of about 0.1 $\mu$M to about 2.5 $\mu$M, and may be outside the range.

On the other hand, the dissociation constant ($K_d$) of the boron-containing compound represented by Formula 2 and lysine, which is the monomeric amino acid, is about several mM, and the boron-containing compound may have very low binding ability with lysine contained in proteins other than albumin. In fact, although many lysines exist in general proteins, boron-containing compounds represented by Formula 2 were shown to have very low binding ability to proteins other than albumin. The fact that boron-containing compounds represented by Formula 2 bind particularly strongly to lysine-199 present in HSA suggests that, the overall structure of the compounds of Formula 2 and the spatial interaction of

the SS1 site of HSA are suitable in addition to the covalent bonding with lysine. That is, it can be seen that the binding ability of the compound of Formula 2 to HSA is strong due to the combination of the binding force through interactions by van der Waals bonds and hydrogen bonds with the SS1 site and the binding force by covalent bonds with lysine.

**Evaluation Example 2 (Quantum Yield Evaluation)**

The quantum yield (Φ) of each of albumin conjugate B-A1 to albumin conjugate B-A5 was calculated with reference to Angew. Chem. Int. ed. 2016, 55, 14728-14732. Specifically, fluorescein, known to have a quantum yield of about 79.0% in an ethanol solution, was used as a reference sample, and each of the albumin conjugates was used as a measurement sample. Results thereof were calculated according to Equation 1 below, and are shown in Table 2 below.

Equation 1

$$\Phi = (\Phi_S) \times (I \times Ab_S \times \eta^2) / (I_S \times Ab \times (\eta_S)^2)$$

In Equation 1,

Φ is the quantum yield of the measurement sample,
$\Phi_S$ is the quantum yield of the reference sample,
Ab is the absorbance in the absorption spectrum of the measured sample,
Abs is the absorbance in the absorption spectrum of the reference sample,
I is the integration area of the emission spectrum of the measurement sample,
Is is the integrated area of the emission spectrum of the reference sample,
η is the refractive index of the measured sample, and
$\eta_S$ is the refractive index of the measured sample.

[Table 2]

| Boron-containing compound | B1 | B2 | B3 | B4 | B5 |
|---|---|---|---|---|---|
| Albumin conjugate | B-A1 | B-A2 | B-A3 | B-A4 | B-A5 |
| Quantum yield (%) | 52.6 | 5.6 | ND | 3.3 | 91.1 |

In Table 2, the quantum yield of boron-containing compound B3 was relatively low and was not measured (not detecting; ND).

From Table 2 above, it can be seen that albumin conjugate B-A1 and albumin conjugate B-A5 have relatively high quantum yields and therefore excellent fluorescence properties. Therefore, the location of the cancer can be visually identified by albumin conjugate B-A1 and albumin conjugate B-A5 selectively accumulated in the cancer (cancer cells and/or cancer tissue). That is, the albumin conjugate according to an embodiment of the present disclosure can perform fluorescence imaging of the location of cancer.

**Evaluation Example 3-1 (ROS Generation Evaluation)**

2',7'-Dichlorodihydrofluorescein diacetate (DCFH-DA) generates fluorescence in proportion to the amount of reactive oxygen species (ROS) generated.

10 μM albumin conjugate B-A1-HSA and 5 μM DCFH-DA were mixed in PBS buffer solution (pH 7.4) and irradiated with light at 530 nm. The fluorescence generated over time was measured using a Scinco FS-2, and results thereof are shown in FIGS. 3A and 6. In FIG. 6, "F.I. - F.$I_0$." stands for "fluorescence intensity - initial fluorescence intensity." Albumin conjugate B-A1-HSA refers to albumin conjugate B-A1 produced by reacting albumin with a boron-containing compound B1, wherein the albumin is HSA.

The results using albumin conjugate B-A2-HSA instead of albumin conjugate B-A1-HSA are shown in FIGS. 3B and 6. Albumin conjugate B-A2-HSA refers to albumin conjugate B-A2 produced by reacting albumin with a boron-containing compound B2, wherein the albumin is HSA.

The results obtained using albumin conjugate B-A3-HSA instead of albumin conjugate B-A1-HSA are shown in FIGS. 3C and 6. Albumin conjugate B-A3-HSA refers to albumin conjugate B-A3 produced by reacting albumin with a boron-containing compound B3, wherein the albumin is HSA.

The results obtained using boron-containing compound B1 instead of albumin conjugate B-A1-HSA are shown in FIGS. 4A and 6.

The results obtained using boron-containing compound B2 instead of albumin conjugate B-A1-HSA are shown in FIGS. 4B and 6.

The results obtained using boron-containing compound B3 instead of albumin conjugate B-A1-HSA are shown in FIGS. 4C and 6.

The results obtained using DCFH-DA without using albumin conjugate B-A1-HSA are shown in FIGS. 5A and 6.

The results obtained using HSA without using albumin conjugate B-A1-HSA and DCFH-DA are shown in FIGS. 5B and 6.

Referring to FIGS. 3A to 3C, 4A to 4C, 5A and 5B, it can be seen that the amount of reactive oxygen species produced increases when the albumin conjugate B-A2-HSA according to an embodiment of the present disclosure was used, compared to when only a boron-containing compound (B1, B2 or B3) was used, only DCFH-DA was used, or only HSA was used. In particular, it can be seen that albumin conjugate B-A2-HSA has an excellent ability to generate reactive oxygen species (ROS) when irradiated with light of about 520 nm to about 540 nm. However, the reactive oxygen species (ROS) generating ability of the albumin conjugate according to Formula 1 is not limited to being excellent only when irradiated with light of about 520 nm to about 540 nm, and the reactive oxygen species (ROS) generating ability of the albumin conjugate according to Formula 1 may be excellent even when irradiated with light having any wavelength such as ultraviolet light, visible light, near-infrared light, or infrared light.

Referring to FIG. 6 and Table 2, it can be seen that albumin conjugate B-A2-HSA has a relatively low quantum yield but has excellent reactive oxygen species generation ability.

Since the albumin conjugate contains tetra-coordinate boron and the boron-containing compound contains tri-coordinate boron, the ability of the albumin conjugate to generate reactive oxygen species may be superior to that of the boron-containing compound to generate reactive oxygen species. That is, a difference in reactive oxygen species generation ability occurs before and after the boron-containing compound reacts (or binds) with albumin to produce the albumin conjugate.

However, referring to FIGS. 3C, 4C, and 5B, the reactive oxygen species generating ability of the albumin conjugate B-A3-HSA may seem relatively similar to the reactive oxygen species generating ability of the boron-containing compound B3 or the reactive oxygen species generating ability of HSA, and therefore, Evaluation Example 3-2 was performed.

## Evaluation Example 3-2 (Evaluation of ROS production according to lysine concentration)

i) 1.0 mM lysine (hereinafter, Lys) was mixed in PBS buffer solution (pH 7.4) and irradiated with light of about 530 nm. The fluorescence generated over time was measured using a Scinco FS-2, and results thereof are shown in FIG. 7A. ii) The result of mixing 10 μM boron-containing compound B3 instead of the 1.0 mM lysine (Lys) is shown in FIG. 7B, iii) the result of mixing 10 μM boron-containing compound B3 and 0.1 mM lysine (Lys) instead of the 1.0 mM lysine (Lys) is shown in FIG. 7C, and iv) the result of mixing 10 μM boron-containing compound B3 and 1.0 mM lysine (Lys) instead of the 1.0 mM lysine (Lys) is shown in FIG. 7D.

Referring to FIGS. 7A to 7D, it can be seen that when boron-containing compound B3 is mixed with a relatively large concentration of monomeric lysine (e.g., about 1.0 mM), a relatively large amount of reactive oxygen species is generated.

Since cancer cells and/or cancer tissues receive nutrients necessary for growth through albumin, albumin conjugate B-A3 may be decomposed within cancer cells to produce boron-containing compound B3. Since cancer cells contain about 1.0 mM or more of monomeric lysine and amino acids, the generated boron-containing compound B3 may be mixed with monomeric lysine and amino acids to generate a relatively large amount of reactive oxygen species. That is, the albumin conjugate B-A3 (or boron-containing compound B3) accumulated in cancer cells is expected to be able to photodynamically treat cancer.

## Evaluation Example 4 (Cancer Target Evaluation)

Cancer cells (MDA-MB-231 cells) were transplanted into six mice, and it was confirmed that the cancer tissues had grown to about 100-150 $mm^3$ after about 7-10 days. Thereafter, i) a vehicle, ii) albumin conjugate B-A2-BSA (50 μM) produced by reacting boron-containing compound B2 with bovine serum albumin (BSA), and iii) albumin conjugate B-A2-HSA (50 μM) produced by reacting boron-containing compound B2 with HSA were intravenously injected into mice. Organ tissues from the mouse were extracted at regular intervals and their fluorescence characteristics were observed, and

results thereof are shown in FIG. 8. Specifically, the fluorescence characteristics of each of the mouse's heart, lung, liver, kidney, spleen, and cancer tissues were observed.

Nanomaterials may selectively accumulate in cancer tissues by the enhanced permeability and retention (EPR) effect. However, in general, nanomaterials may selectively accumulate in the liver, which may cause side effects such as hepatotoxicity.

Referring to FIG. 8, it can be seen that B-A2-HSA selectively accumulates only in cancer tissues with excellent efficiency and relatively does not accumulate in the liver. This may mean that B-A2-HSA is not simply accumulated in cancer tissues by the EPR effect, but rather selectively accumulates only in cancer tissues due to the ability of cancer tissues to accept albumin. Therefore, B-A2-HSA may reduce the possibility of accumulating in the liver and causing side effects.

Evaluation Example 4 was conducted on boron-containing compound B2, but since the boron-containing compound represented by Formula 2 can be produced as an albumin conjugate represented by Formula 1 by Reaction Scheme 1, it is obvious that the albumin conjugate according to the present disclosure can be selectively accumulated in cancer tissues.

In addition, since the albumin conjugate represented by Formula 1 and the boron-containing compound represented by Formula 2 can selectively accumulate in cancer tissues, it is obvious that when an anticancer agent is bound to the albumin conjugate or the boron-containing compound, the anticancer agent can be effectively selectively accumulated in (or transported to) cancer tissues.

## Evaluation Example 5 (Cancer Treatment Evaluation 1)

Cancer (MDA-MB-231 cells) were transplanted into six mice, and it was confirmed that the cancer tissue grew to about 100 $mm^3$ or more.

i) The first subject is the control group.
ii) Boron-containing compound B2 was intravenously injected (I.V.) into the second subject. (B2(I.V.))
iii) Albumin conjugate B-A2-HSA produced by reacting boron-containing compound B2 with HSA was intravenously injected into the third subject. (B-A2-HSA(I.V.))
iv) B-A2-HSA was directly injected into cancer tissue (intratumor, I.T.) in the fourth subject. (B-A2-HSA(I.T.))
v) Albumin conjugate B-A2-BSA produced by reacting boron-containing compound B2 with bovine serum albumin (BSA) was intravenously injected into the fifth subject. (B-A2-BSA(I.V.))
vi) B-A2-BSA was directly injected into cancer tissue in the sixth subject. (B-A2-BSA(I.T.))

Four hours after injection, green light (about 490 to 530 nm) was irradiated for 20 minutes. The injections and green light irradiation were performed once a week, for a total of three times. The size of the cancer tissue of each mouse was compared 28 days after the cancer transplantation, and results are shown in FIGS. 9A and 9B.

Referring to FIGS. 9A and 9B, it can be confirmed that albumin conjugates (B-A2-HSA, B-A2-BSA) according to the present disclosure can reduce the size of cancer tissue regardless of whether they were intravenously injected or directly injected into cancer tissues.

Since mice do not contain HSA, the cancer treatment effect may be superior when injected with albumin conjugates (B-A2-HSA, B-A2-BSA) than when injected with boron-containing compound B2. However, it is obvious that injection of boron-containing compound B2 can have an excellent cancer treatment effect in subjects other than mice, including HSA.

## Evaluation Example 6 (Cancer Treatment Evaluation 2)

Cancer (MDA-MB-231 cells) were transplanted into eight mice, and it was confirmed that the cancer tissues grew to about 100 $mm^3$ or more.

i) Each of the first subject and the fifth subject is a control group.
ii) Boron-containing compound B2 was intravenously injected into each of the second subject and the sixth subject.
iii) Albumin conjugate B-A2-HSA produced by reacting boron-containing compound B2 with HSA was intravenously injected into each of the third subject and the seventh subject.
iv) Albumin conjugate B-A2-BSA produced by reacting boron-containing compound B2 with bovine serum albumin (BSA) was intravenously injected into each of the fourth subject and the eighth subject.

Four hours after the intravenous injection, green light (about 490 to 530 nm) was irradiated to each of the first to fourth subjects for 20 minutes, and no light was irradiated to the fifth to eighth subjects. The injections and green light

irradiation were performed once a week, for a total of three times.

The size of cancer tissue in each mouse was compared 28 days after the time of cancer transplantation, and results are shown in FIG. 10A to 10C. Specifically, FIG. 10A is a diagram showing the results of staining cancer in each of a first subject to an eighth subject, FIG. 10B is a diagram showing the excised cancer of each of the first subject to a fourth subject, and FIG. 10C is a diagram showing the size of cancer in each of the first subject to the fourth subject over time.

Referring to FIGS. 10A to 10C, it can be seen that the albumin conjugates (B-A2-HSA, B-A2-BSA) according to the present disclosure can effectively reduce the size of cancer tissue under light irradiation.

## Claims

1. An albumin conjugate represented by Formula 1:

Formula 1

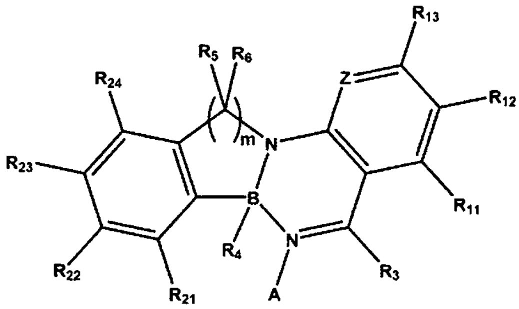

**wherein,** in Formula 1,

A is a group other than an amine group of albumin,
Z is N, O, or $C(R_{14})$,
$R_{11}$ to $R_{14}$, $R_{21}$ to $R_{24}$ and $R_3$ to $R_6$ are each independently selected from hydrogen, deuterium, -F, -Cl, -Br, -I, a hydroxyl group, a cyano group, a nitro group, a formyl group, a carboxyl group, an amidino group, a hydrazino group, a hydrazono group, a substituted or unsubstituted $C_1$-$C_{10}$ alkyl group, a substituted or unsubstituted $C_2$-$C_{10}$ alkenyl group, a substituted or unsubstituted $C_2$-$C_{10}$ alkynyl group, a substituted or unsubstituted $C_1$-$C_{20}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{20}$ aryl group, a substituted or unsubstituted $C_6$-$C_{20}$ aryloxy group, a substituted or unsubstituted $C_6$-$C_{20}$ arylthio group, a substituted or unsubstituted $C_1$-$C_{20}$ heteroaryl group, $-Si(Q_1)(Q_2)(Q_3)$, $-N(Q_1)(Q_2)$, $-B(Q_1)(Q_2)$, $-C(=O)(Q_1)$, $-S(=O)_2(Q_1)$, and $-P(=O)(Q_1)(Q_2)$,
optionally, i) $R_{11}$ and $R_{12}$, ii) $R_{12}$ and $R_{13}$, iii) $R_{13}$ and $R_{14}$, iv) $R_{21}$ and $R_{22}$, v) $R_{22}$ and $R_{23}$, vi) $R_{23}$ and $R_{24}$, or vii) any combination thereof are bound to each other to form a substituted or unsubstituted $C_3$-$C_{10}$ carbocyclic group or a substituted or unsubstituted $C_1$-$C_{10}$ heterocyclic group,
m is an integer from 1 to 5, and
$Q_1$ to $Q_3$ are each independently selected from:

hydrogen, deuterium, -F, -Cl, -Br, -I, a hydroxyl group, a cyano group, a nitro group, an amidino group, a hydrazino group, and a hydrazono group; and
a $C_1$-$C_{10}$ alkyl group, a $C_2$-$C_{10}$ alkenyl group, a $C_2$-$C_{10}$ alkynyl group, a $C_1$-$C_{10}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{20}$ aryl group, and a $C_1$-$C_{20}$ heteroaryl group, each unsubstituted or substituted with hydrogen, deuterium, -F, -Cl, -Br, -I, a hydroxyl group, a cyano group, a nitro group, an amidino group, a hydrazino group, a hydrazono group, or any combination thereof.

2. The albumin conjugate of claim 1, wherein
the amine group is an amine group contained in lysine.

3. The albumin conjugate of claim 1, wherein
the amine group is located at an SS1 site of the albumin.

4. The albumin conjugate of claim 1, wherein the albumin conjugate includes human serum albumin or bovine serum albumin.

5. The albumin conjugate of claim 1, wherein in Formula 1, m is 1.

6. The albumin conjugate of claim 1, wherein the albumin conjugate has fluorescent properties.

7. The albumin conjugate of claim 1, wherein the albumin conjugate generates reactive oxygen species under light irradiation.

8. A method of producing an albumin conjugate, the method comprising reacting albumin with a boron-containing compound represented by Formula 2:

Formula 2

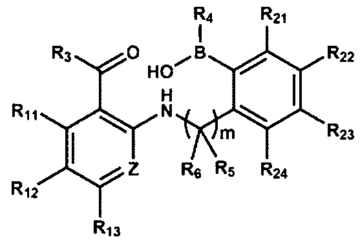

**wherein,** in Formula 2,

Z is N, O, or C($R_{14}$),
$R_{11}$ to $R_{14}$, $R_{21}$ to $R_{24}$ and $R_3$ to $R_6$ are each independently selected from hydrogen, deuterium, -F, -Cl, -Br, -I, a hydroxyl group, a cyano group, a nitro group, a formyl group, a carboxyl group, an amidino group, a hydrazino group, a hydrazono group, a substituted or unsubstituted $C_1$-$C_{10}$ alkyl group, a substituted or unsubstituted $C_2$-$C_{10}$ alkenyl group, a substituted or unsubstituted $C_2$-$C_{10}$ alkynyl group, a substituted or unsubstituted $C_1$-$C_{20}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{20}$ aryl group, a substituted or unsubstituted $C_6$-$C_{20}$ aryloxy group, a substituted or unsubstituted $C_6$-$C_{20}$ arylthio group, a substituted or unsubstituted $C_1$-$C_{20}$ heteroaryl group, -Si($Q_1$)($Q_2$)($Q_3$), - N($Q_1$)($Q_2$), -B($Q_1$)($Q_2$), -C(=O)($Q_1$), -S(=O)$_2$($Q_1$), and -P(=O)($Q_1$)($Q_2$),
optionally, i) $R_{11}$ and $R_{12}$, ii) $R_{12}$ and $R_{13}$, iii) $R_{13}$ and $R_{14}$, iv) $R_{21}$ and $R_{22}$, v) $R_{22}$ and $R_{23}$, vi) $R_{23}$ and $R_{24}$, or vii) any combination thereof are bound to each other to form a substituted or unsubstituted $C_3$-$C_{10}$ carbocyclic group or a substituted or unsubstituted $C_1$-$C_{10}$ heterocyclic group,
m is an integer from 1 to 5, and
$Q_1$ to $Q_3$ are each independently selected from:
hydrogen, deuterium, -F, -Cl, -Br, -I, a hydroxyl group, a cyano group, a nitro group, an amidino group, a hydrazino group, and a hydrazono group; and
a $C_1$-$C_{10}$ alkyl group, a $C_2$-$C_{10}$ alkenyl group, a $C_2$-$C_{10}$ alkynyl group, a $C_1$-$C_{10}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{20}$ aryl group, and a $C_1$-$C_{20}$ heteroaryl group, each unsubstituted or substituted with hydrogen, deuterium, -F, -Cl, -Br, -I, a hydroxyl group, a cyano group, a nitro group, an amidino group, a hydrazino group, a hydrazono group, or any combination thereof.

9. The method of claim 8, wherein the reacting of the boron-containing compound with albumin is performed outside a body of a subject.

10. The method of claim 8, wherein the reacting of the boron-containing compound with albumin is performed in blood of a subject.

11. A composition for targeting, diagnosing or treating cancer, the composition comprising the albumin conjugate of claim

1.

**12.** A composition for targeting, diagnosing or treating cancer, the composition comprising the boron-containing compound of claim 8.

**13.** The composition of claim 11, wherein the albumin conjugate has fluorescent properties.

**14.** The composition of claim 11, wherein the albumin conjugate has a reactive oxygen species-generating property.

FIG. 1

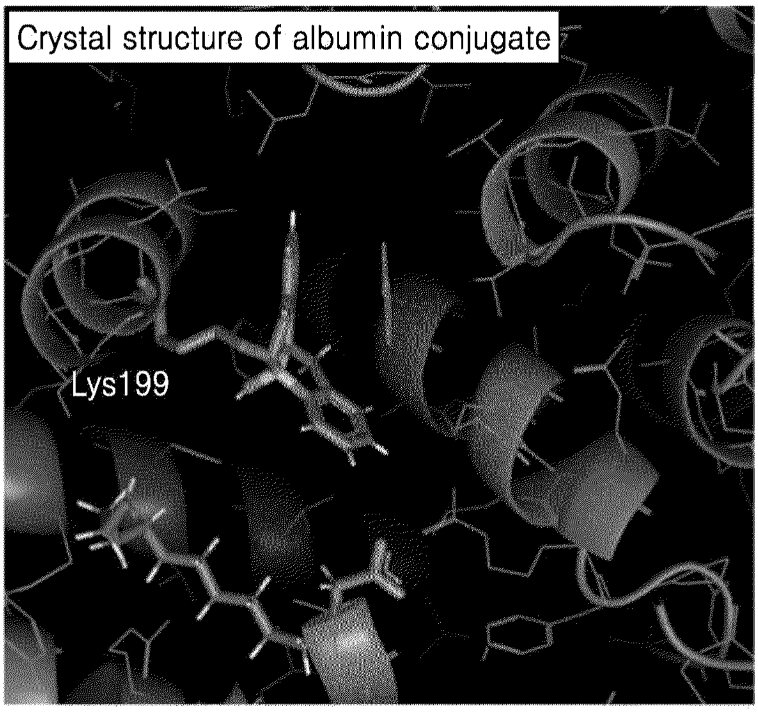

# FIG. 2

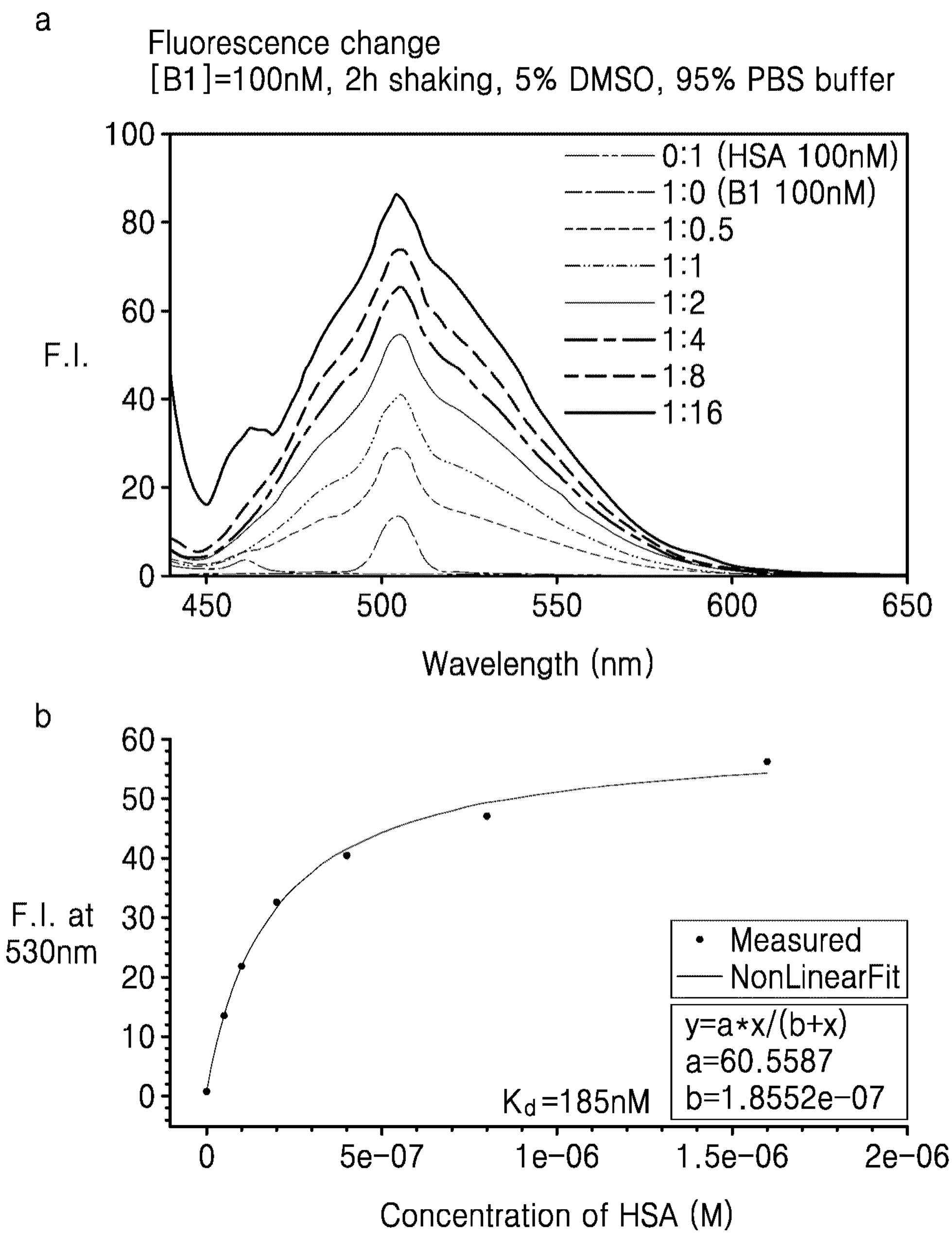
a
Fluorescence change
[B1]=100nM, 2h shaking, 5% DMSO, 95% PBS buffer
0:1 (HSA 100nM)
1:0 (B1 100nM)
1:0.5
1:1
1:2
1:4
1:8
1:16
F.I.
Wavelength (nm)
b
F.I. at 530nm
Measured
NonLinearFit
y=a*x/(b+x)
a=60.5587
b=1.8552e-07
Kd=185nM
Concentration of HSA (M)

FIG. 3A

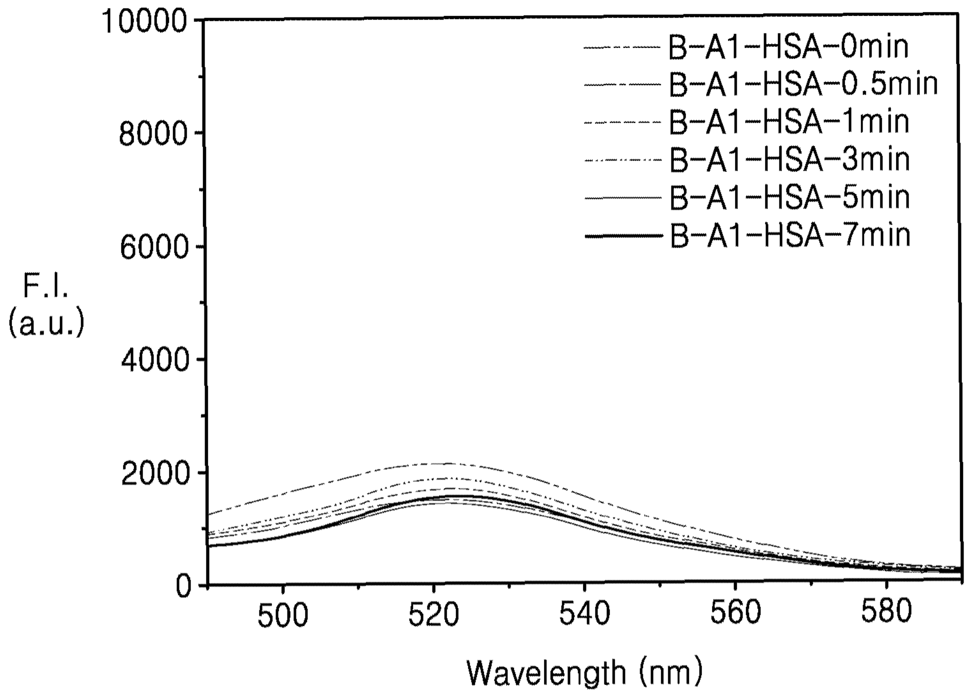
10000
8000
6000
4000
2000
0
F.I.
(a.u.)
500
520
540
560
580
Wavelength (nm)
B-A1-HSA-0min
B-A1-HSA-0.5min
B-A1-HSA-1min
B-A1-HSA-3min
B-A1-HSA-5min
B-A1-HSA-7min

FIG. 3B

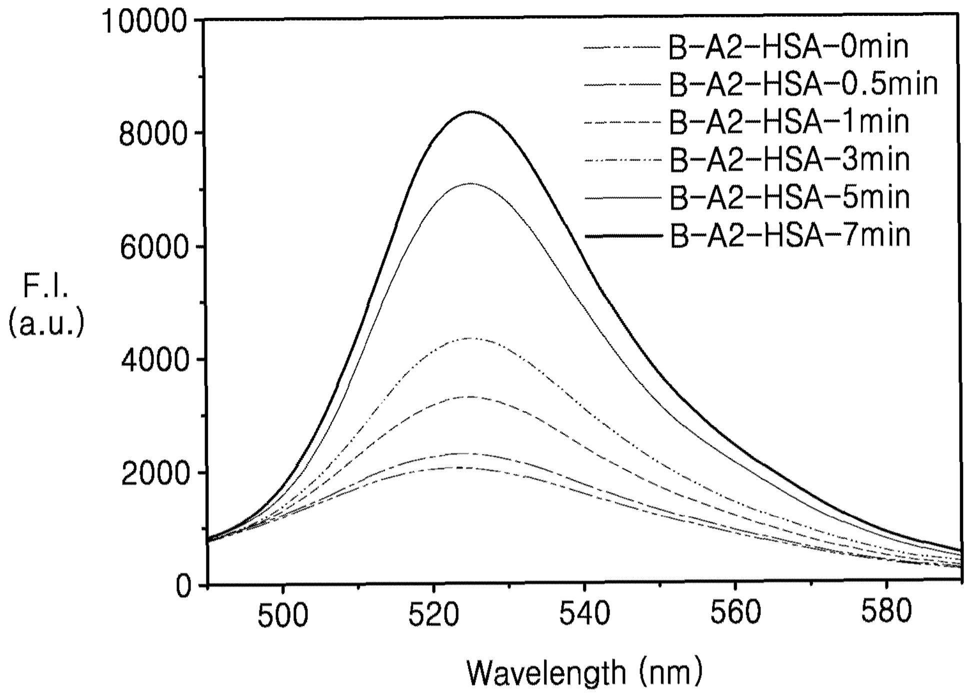

10000
8000
6000
4000
2000
0
F.I.
(a.u.)
500
520
540
560
580
Wavelength (nm)
B-A2-HSA-0min
B-A2-HSA-0.5min
B-A2-HSA-1min
B-A2-HSA-3min
B-A2-HSA-5min
B-A2-HSA-7min

FIG. 3C

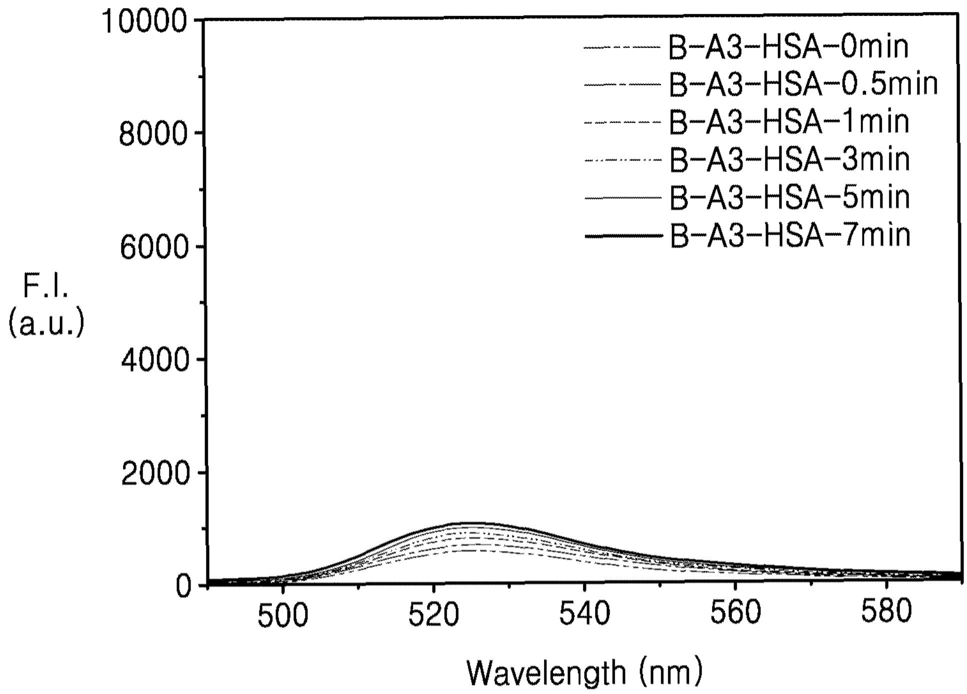
10000
8000
6000
4000
2000
0
F.I.
(a.u.)
500
520
540
560
580
Wavelength (nm)
B-A3-HSA-0min
B-A3-HSA-0.5min
B-A3-HSA-1min
B-A3-HSA-3min
B-A3-HSA-5min
B-A3-HSA-7min

FIG. 4A

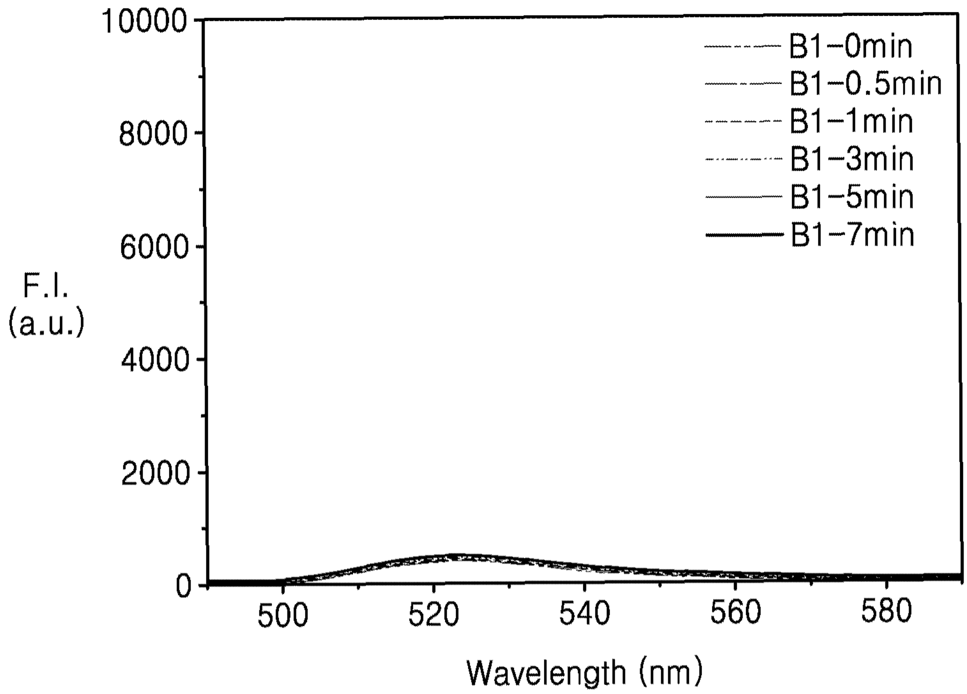
10000
8000
6000
4000
2000
0
F.I.
(a.u.)
500
520
540
560
580
Wavelength (nm)
B1-0min
B1-0.5min
B1-1min
B1-3min
B1-5min
B1-7min

FIG. 4B

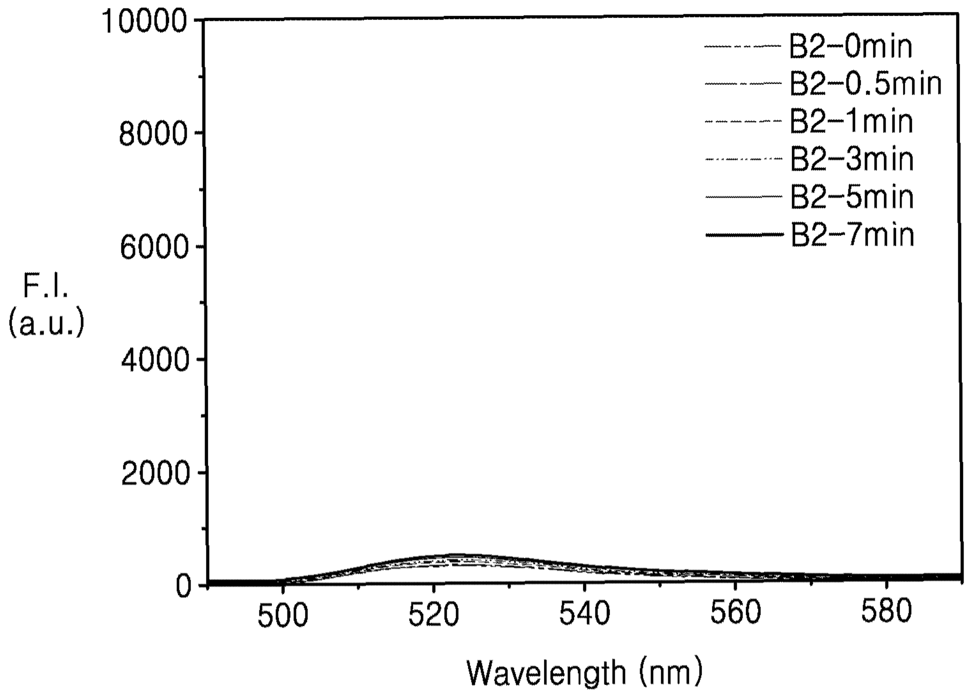
10000
8000
6000
4000
2000
0
F.I.
(a.u.)
500
520
540
560
580
Wavelength (nm)
B2-0min
B2-0.5min
B2-1min
B2-3min
B2-5min
B2-7min

FIG. 4C

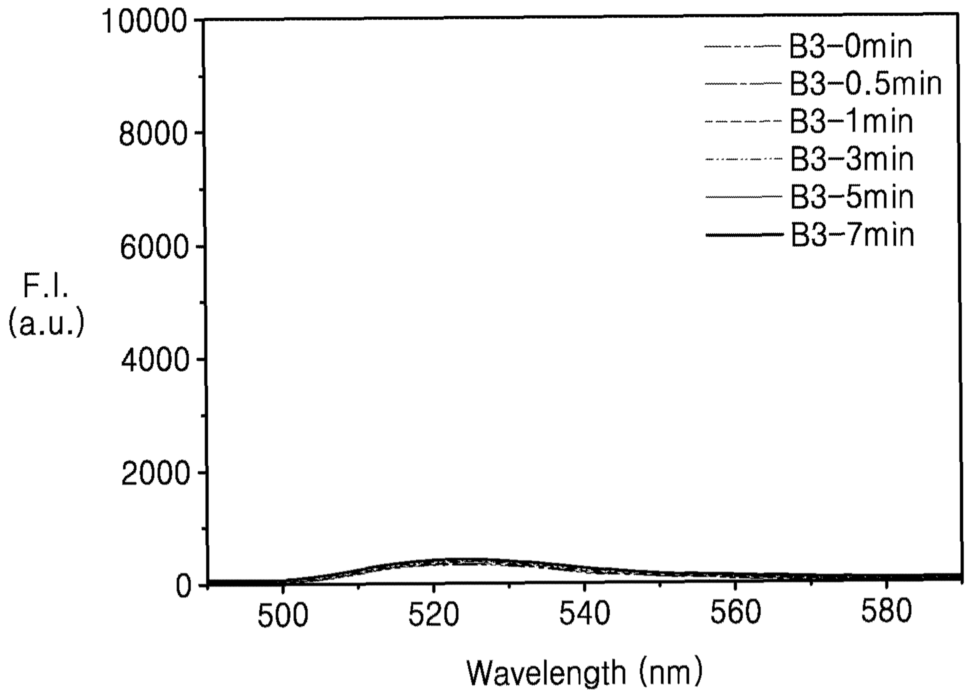
10000
8000
6000
4000
2000
0
F.I.
(a.u.)
500
520
540
560
580
Wavelength (nm)
B3-0min
B3-0.5min
B3-1min
B3-3min
B3-5min
B3-7min

FIG. 5A

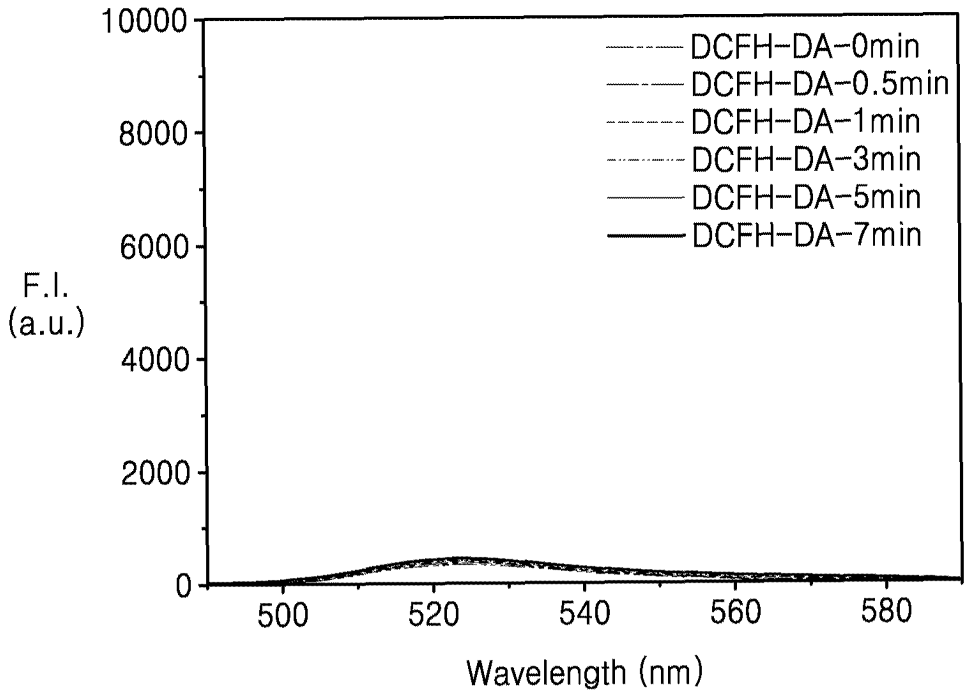
10000
8000
6000
4000
2000
0
F.I.
(a.u.)
500
520
540
560
580
Wavelength (nm)
DCFH-DA-0min
DCFH-DA-0.5min
DCFH-DA-1min
DCFH-DA-3min
DCFH-DA-5min
DCFH-DA-7min

FIG. 5B

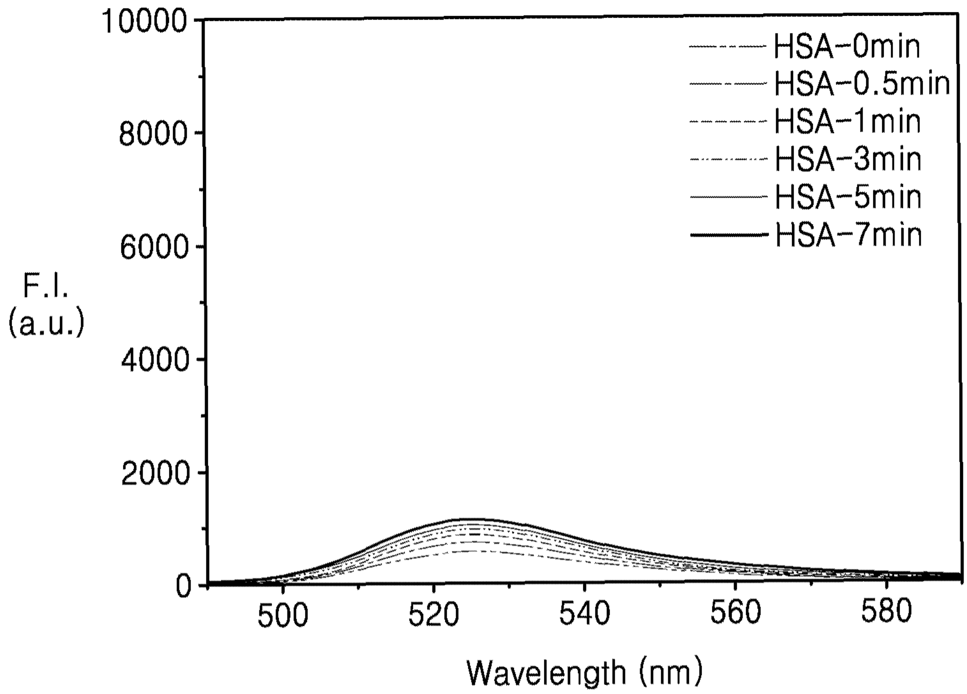
10000
8000
6000
4000
2000
0
F.I.
(a.u.)
HSA-0min
HSA-0.5min
HSA-1min
HSA-3min
HSA-5min
HSA-7min
500
520
540
560
580
Wavelength (nm)

FIG. 6

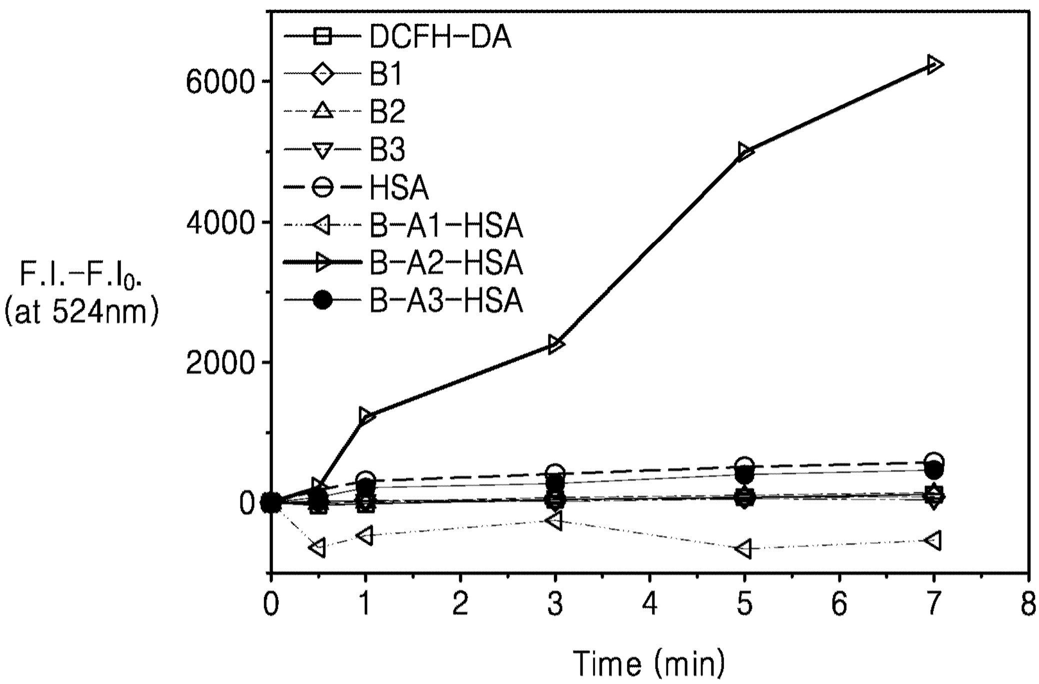
DCFH-DA
B1
B2
B3
HSA
B-A1-HSA
B-A2-HSA
B-A3-HSA
6000
4000
2000
0
F.I.-F.I0.
(at 524nm)
0
1
2
3
4
5
6
7
8
Time (min)

FIG. 7A

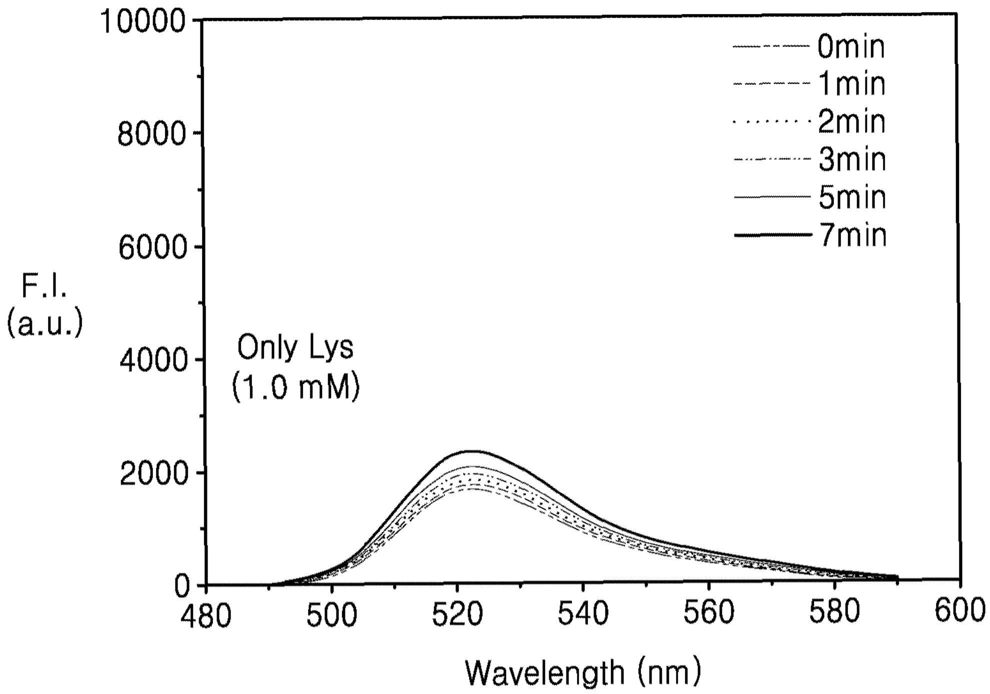

10000
8000
6000
4000
2000
0
F.I.
(a.u.)
0min
1min
2min
3min
5min
7min
Only Lys
(1.0 mM)
480
500
520
540
560
580
600
Wavelength (nm)

FIG. 7B

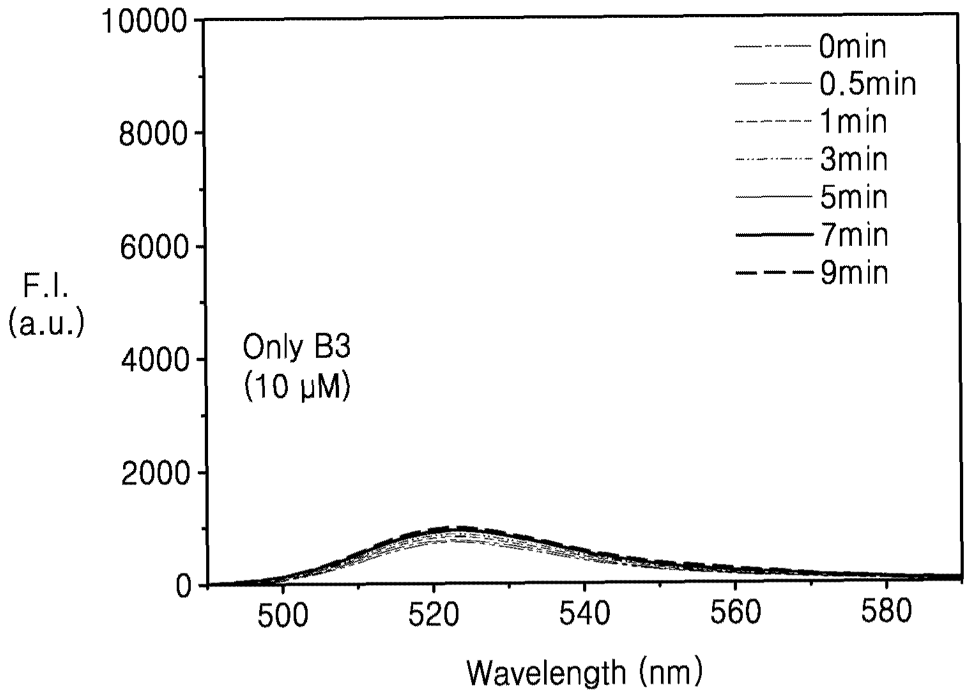
10000
8000
6000
4000
2000
0
F.I.
(a.u.)
Only B3
(10 μM)
0min
0.5min
1min
3min
5min
7min
9min
500
520
540
560
580
Wavelength (nm)

FIG. 7C

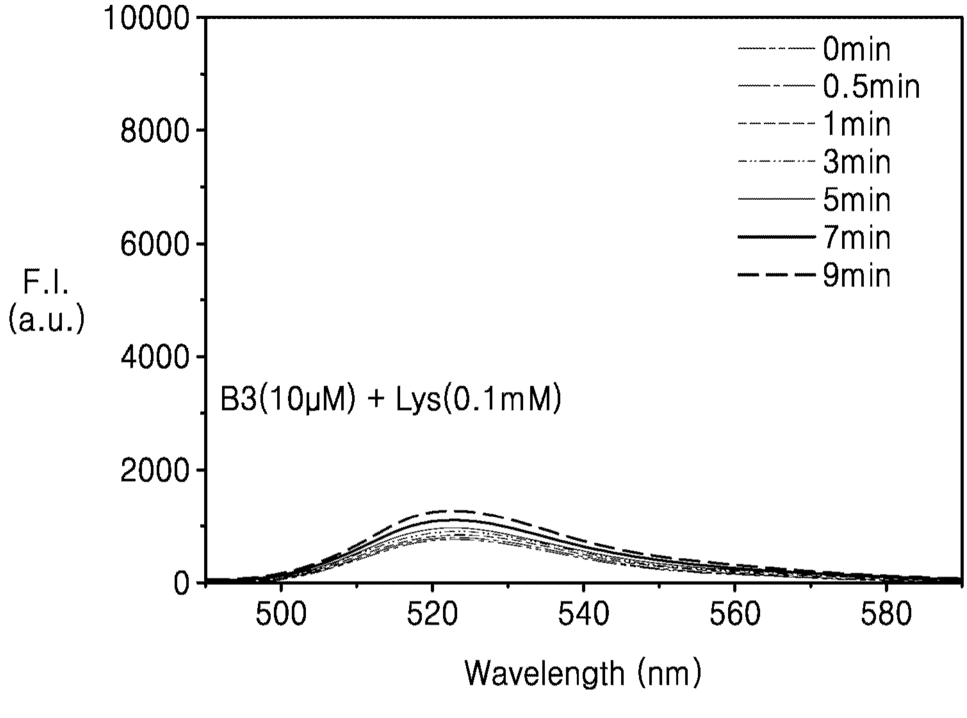
10000
8000
6000
4000
2000
0
F.I.
(a.u.)
0min
0.5min
1min
3min
5min
7min
9min
B3(10μM) + Lys(0.1mM)
500
520
540
560
580
Wavelength (nm)

FIG. 7D

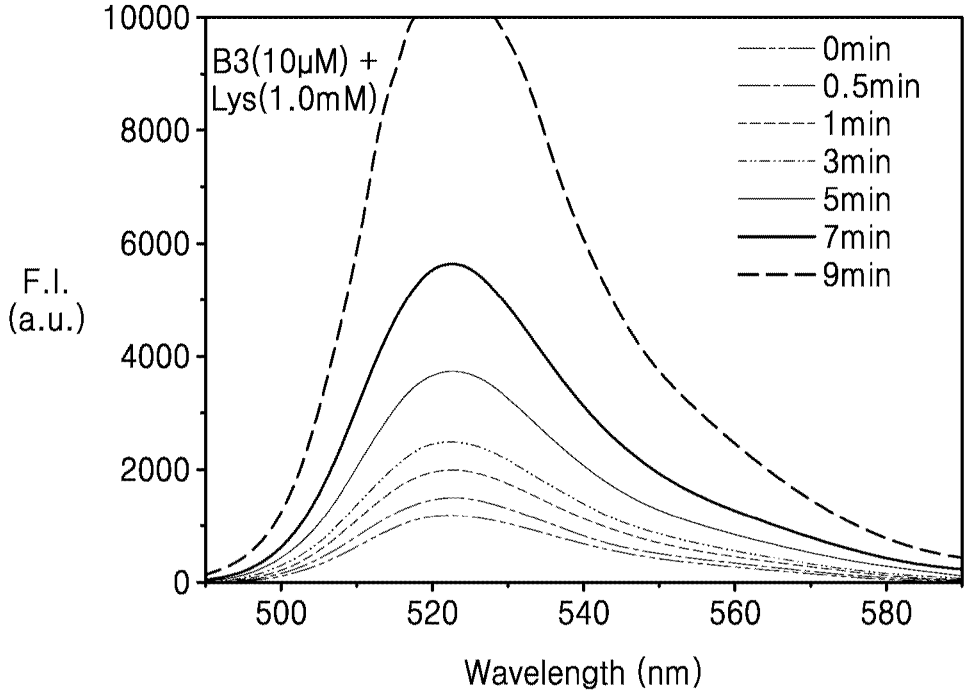
B3(10μM) +
Lys(1.0mM)
0min
0.5min
1min
3min
5min
7min
9min
F.I.
(a.u.)
10000
8000
6000
4000
2000
0
500
520
540
560
580
Wavelength (nm)

FIG. 8

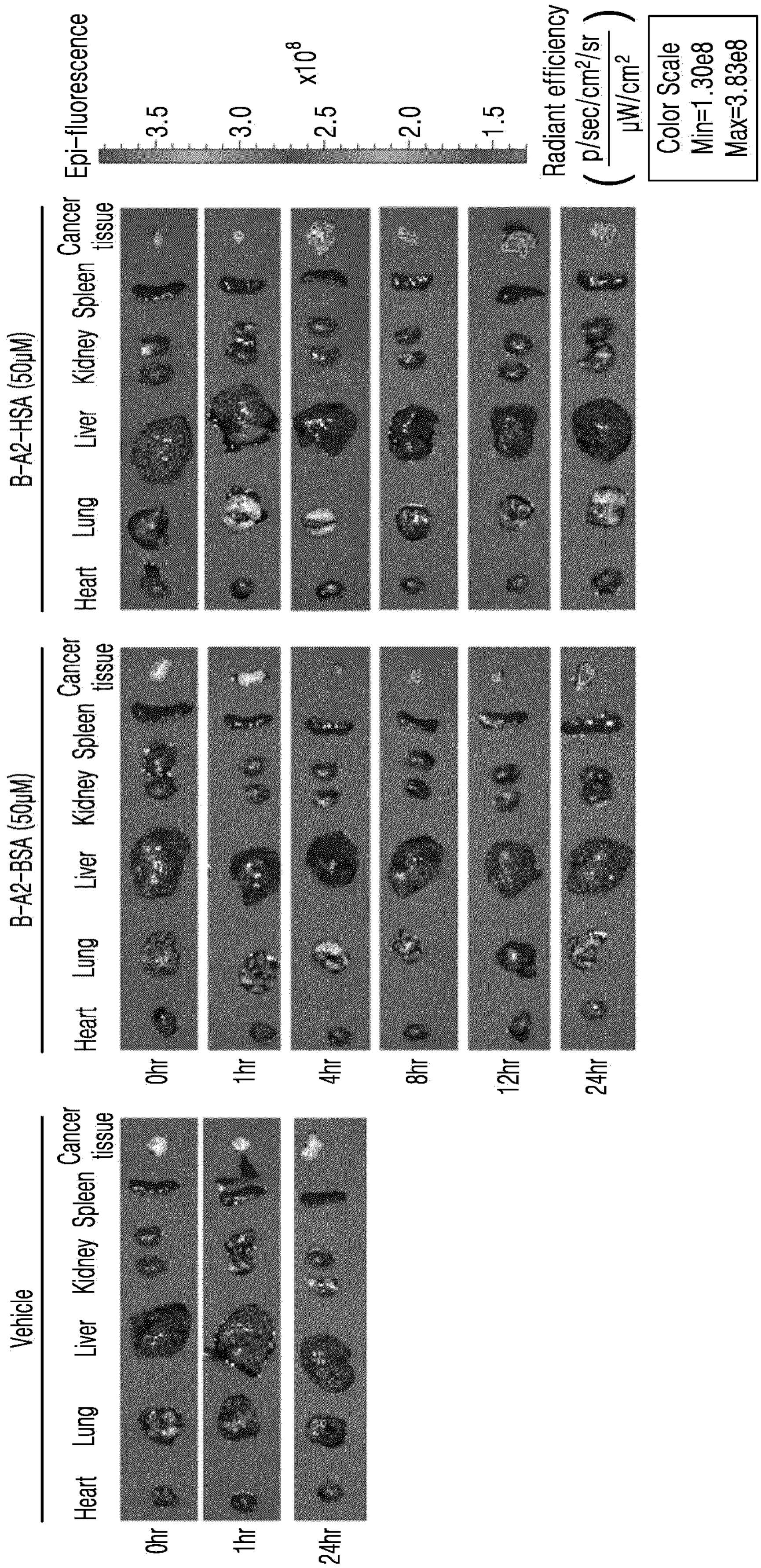
Vehicle
B-A2-BSA (50μM)
B-A2-HSA (50μM)
Heart
Lung
Liver
Kidney
Spleen
Cancer tissue
0hr
1hr
4hr
8hr
12hr
24hr
Epi-fluorescence
3.5
3.0
2.5
2.0
1.5
x10^8
Radiant efficiency
p/sec/cm²/sr
μW/cm²
Color Scale
Min=1.30e8
Max=3.83e8

FIG. 9A

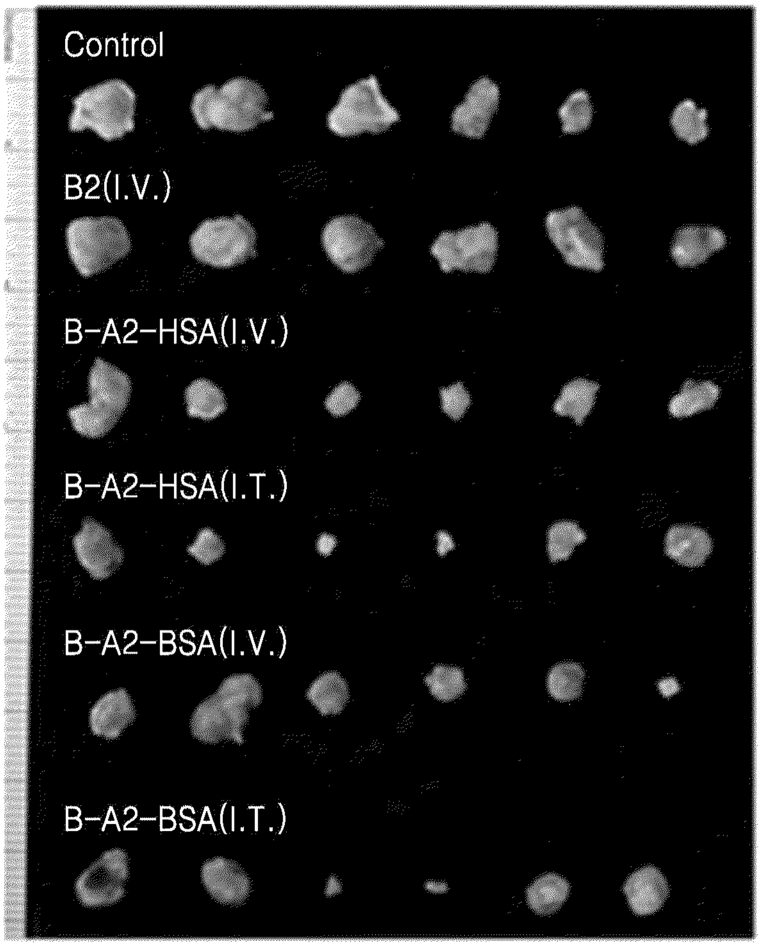

# FIG. 9B

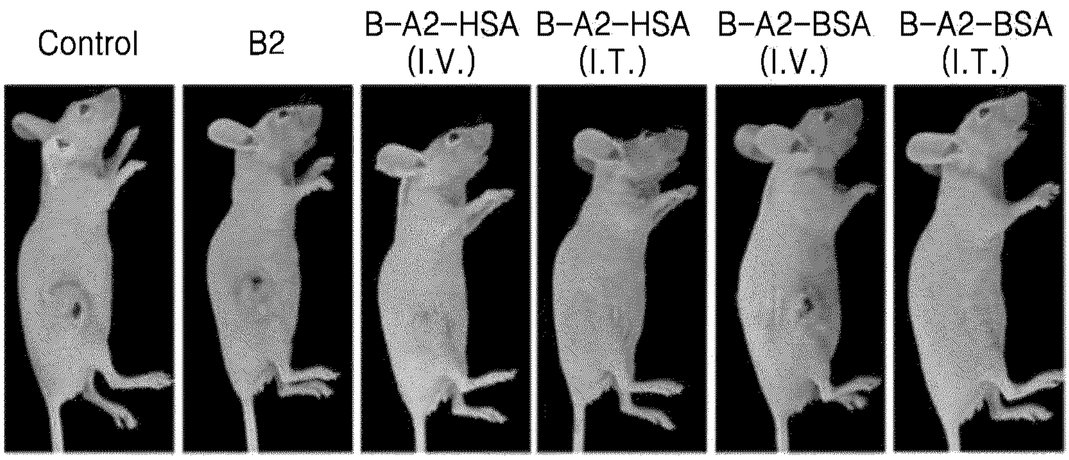

FIG. 10A

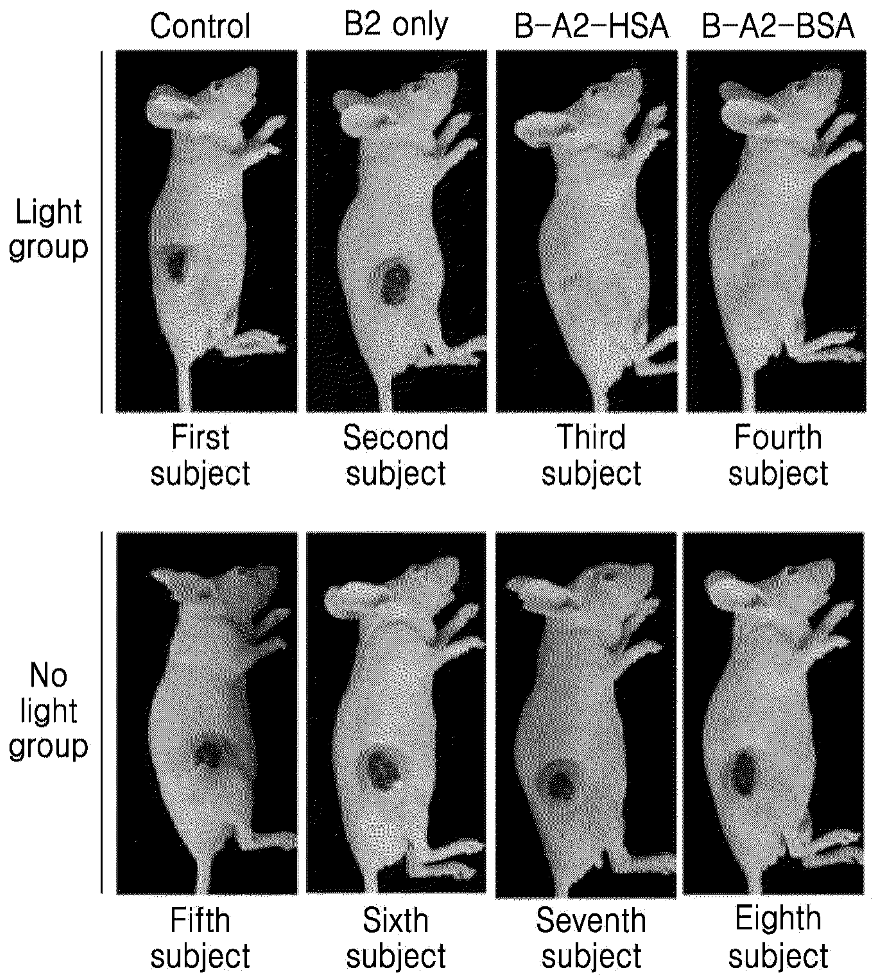
Control
B2 only
B-A2-HSA
B-A2-BSA
Light group
First subject
Second subject
Third subject
Fourth subject
No light group
Fifth subject
Sixth subject
Seventh subject
Eighth subject

# FIG. 10B

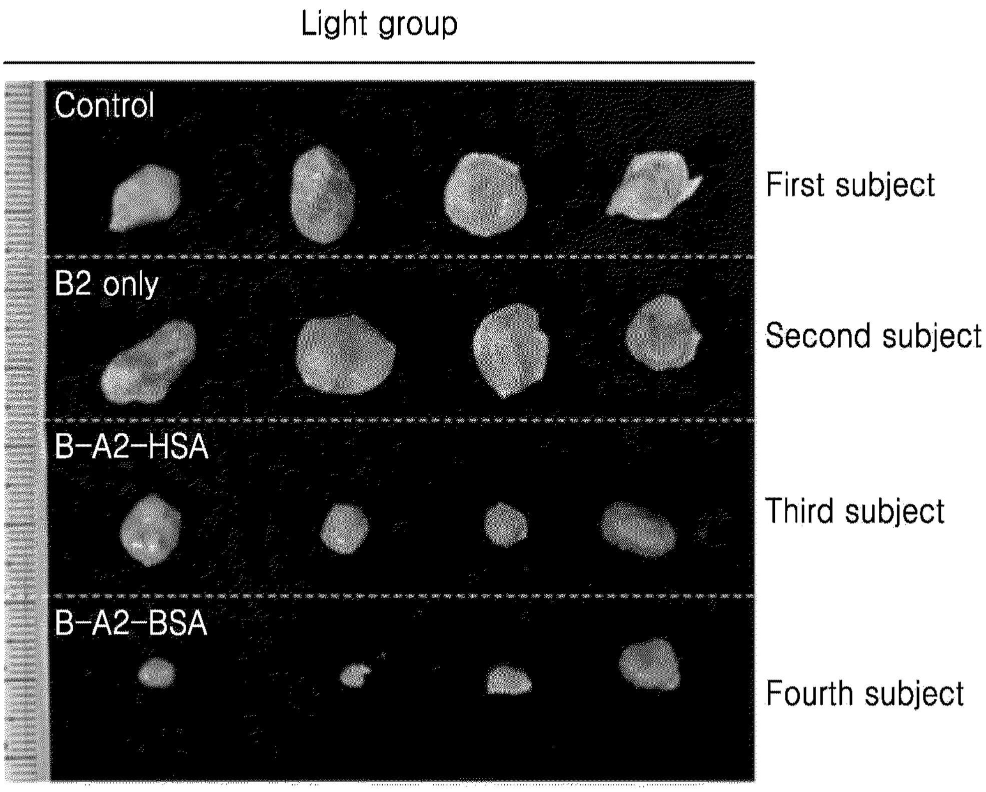
Light group
Control
B2 only
B-A2-HSA
B-A2-BSA
First subject
Second subject
Third subject
Fourth subject

# FIG. 10C

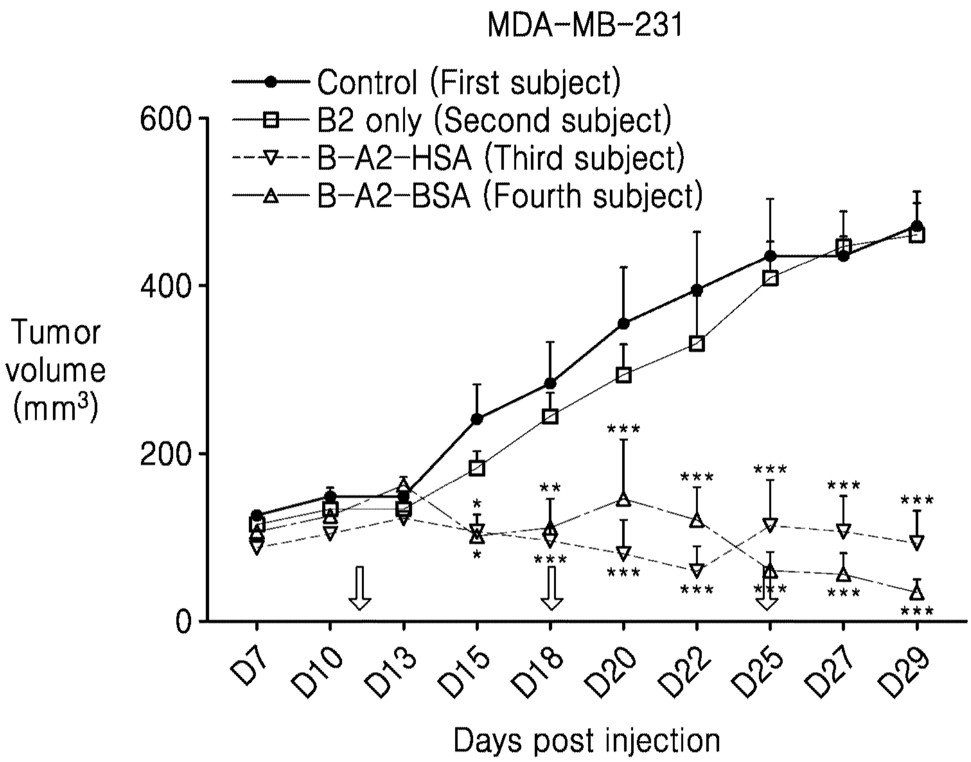


Significance was obtained by Two-way ANOVA test.
All data represents mean ± SEM.
*P<0.05; **P<0.01; ***P<0.001.
⇩Drug administration

**INTERNATIONAL SEARCH REPORT**

International application No. **PCT/KR2024/002350**

**A. CLASSIFICATION OF SUBJECT MATTER**

**A61K 47/64**(2017.01)i; **A61K 49/00**(2006.01)i; **A61K 41/00**(2006.01)i

According to International Patent Classification (IPC) or to both national classification and IPC

**B. FIELDS SEARCHED**

Minimum documentation searched (classification system followed by classification symbols)

A61K 47/64(2017.01); C07F 5/02(2006.01); C09K 11/06(2006.01); C12Q 1/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 알부민(albumin), 아민기(amine group), 붕소 함유 화합물(boron-containing compound), 형광(fluorescence)

**C. DOCUMENTS CONSIDERED TO BE RELEVANT**

| Category* | Citation of document, with indication, where appropriate, of the relevant passages | Relevant to claim No. |
|---|---|---|
| X | KR 10-2413908 B1 (EWHA UNIVERSITY - INDUSTRY COLLABORATION FOUNDATION) 29 June 2022 (2022-06-29) See claims 1, 4 and 6; and paragraphs [0026], [0029], [0056], [0057], [0096]-[0105], [0184] and [0185]. | 9,12 |
| Y | | 1-7,11,13,14 |
| Y | REJA, R. M. et al. Lysine-Targeting Reversible Covalent Inhibitors with Long Residence Time. J. Am. Chem. Soc. 2022, vol. 144, pp. 1152-1157. See figure 3a. | 1-7,11,13,14 |
| A | KR 10-2293339 B1 (EWHA UNIVERSITY - INDUSTRY COLLABORATION FOUNDATION) 25 August 2021 (2021-08-25) See claims 4-7; and paragraphs [0180]-[0182]. | 1-7,9,11-14 |
| A | QUACH, D. et al. Strategic Design of Catalytic Lysine-Targeting Reversible Covalent BCR-ABL Inhibitors. Angewandte Chemie International Edition. 2021, vol. 60, pp. 17131-17137. See abstract; and figure 1(B). | 1-7,9,11-14 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **29 May 2024** | **30 May 2024** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
PCT/KR2024/002350

C. DOCUMENTS CONSIDERED TO BE RELEVANT

| Category* | Citation of document, with indication, where appropriate, of the relevant passages | Relevant to claim No. |
|---|---|---|
| A | SHIRBHATE, M. E. et al. Optical and Fluorescent Dual Sensing of Aminoalcohols by in Situ Generation of BODIPY-like Chromophore. J. Am. Chem. Soc. 2020, vol. 142, pp. 4975-4979. | 1-7,9,11-14 |

Form PCT/ISA/210 (second sheet) (July 2022)

**INTERNATIONAL SEARCH REPORT**

International application No.
**PCT/KR2024/002350**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **8,10**
because they relate to subject matter not required to be searched by this Authority, namely:

Claims 8 and 10 pertain to a method for treatment or diagnosis of the human body by therapy, and thus pertain to a subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2024/002350**

| Patent document cited in search report | Publication date (day/month/year) | Patent family member(s) | Publication date (day/month/year) |
|---|---|---|---|
| KR 10-2413908 B1 | 29 June 2022 | CN 114729927 A | 08 July 2022 |
| | | EP 4057002 A1 | 14 September 2022 |
| | | EP 4057002 A4 | 22 November 2023 |
| | | KR 10-2021-0057700 A | 21 May 2021 |
| | | US 2022-0267670 A1 | 25 August 2022 |
| | | WO 2021-096263 A1 | 20 May 2021 |
| KR 10-2293339 B1 | 25 August 2021 | KR 10-2020-0122259 A | 27 October 2020 |
| | | US 11780857 B2 | 10 October 2023 |
| | | US 2020-0347080 A1 | 05 November 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Angew. Chem. Int.. 2016, vol. 55, 14728-14732